(19) Europäisches Patentamt
European Patent Office
Office européen des brevets

(11) EP 1 947 089 A1

(12) **EUROPEAN PATENT APPLICATION**

(43) Date of publication:
**23.07.2008 Bulletin 2008/30**

(51) Int Cl.:
***C07D 231/06*** *(2006.01)* ***A61K 31/4155*** *(2006.01)*
***A61P 3/04*** *(2006.01)* ***A61P 3/10*** *(2006.01)*

(21) Application number: **07001041.8**

(22) Date of filing: **18.01.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Laboratorios del Dr. Esteve S.A.**
**08041 Barcelona (ES)**

(72) Inventors:
- **Buschmann, Helmut, H., Dr.**
  **08960 Sant Just Desvern (ES)**
- **Torrens-Jover, Antonio**
  **08221 Terrassa (Barcelona) (ES)**
- **Yenes-Minguez, Susana**
  **08750 Molins de Rei (Barcelona) (ES)**
- **Benet-Buchholz, Jordi**
  **43893 Altafulla (Tarragona) (ES)**
- **Sola-Carandell, Lluis**
  **43893 Altafulla (Tarragona) (ES)**

(74) Representative: **Peters, Hajo et al**
**Bosch Graf von Stosch Jehle**
**Patentanwaltsgesellschaft mbH**
**Flüggenstrasse 13**
**80639 München (DE)**

# (54) A-polymorph of a substituted pyrazoline, its preparation and use as medicaments

(57) The present invention relates to the α-Polymorph of (rac)-N-piperidinyl-5-(4-chlorophenyl)-1-(2,4-dichlorophenyl)-4,5-dihydro-1H-pyrazole-3-carboxamide, methods for its preparation, medicaments comprising this compound as well as their use for the preparation of a medicament for the treatment of humans and animals.

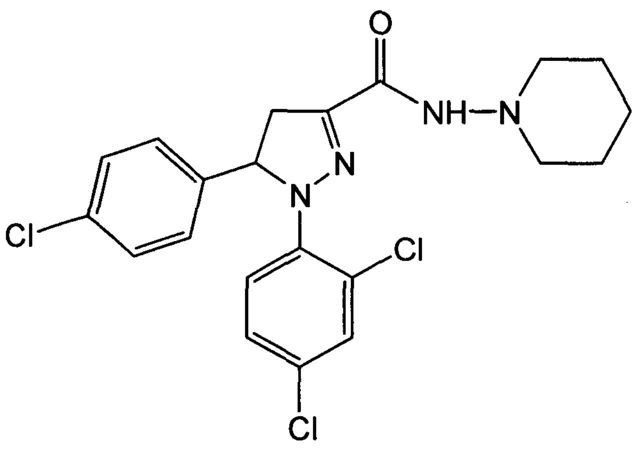



Printed by Jouve, 75001 PARIS (FR)

## Description

The present invention relates to a polymorph of a substituted pyrazoline, methods for its preparation, medicaments comprising the compound as well as use for the preparation of a medicament for the treatment of humans and animals.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide novel compounds for use as active substances in medicaments. In particular, these active substances should be suitable for the modulation of Cannabinoid receptors, more particularly for the modulation of Cannabinoid 1 ($CB_1$) receptors.

Said object was achieved by providing the $\alpha$-polymorph of the piperidinyl-pyrazoline compound given below.

It has been found that these compounds have a high affinity for cannabinoid receptors, particularly for the $CB_1$-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Thus, in one of its aspects the present invention relates to the $\alpha$-Polymorph of (rac)-N-piperidinyl-5-(4-chlorophenyl)-1-(2,4-dichlorophenyl)-4,5-dihydro-1 H-pyrazole-3-carboxamide.

This $\alpha$-Polymorph of (rac)-N-piperidinyl-5-(4-chlorophenyl)-1-(2,4-dichlorophenyl)-4,5-dihydro-1 H-pyrazole-3-carboxamide crystallizes very well and shows a remarkable stability under certain conditions and/or over time e.g. apparently not transforming to any other crystalline phase or to an amorphous solid. It also seems to show a relatively good solubility and also heat resistance. In addition and especially it was resistant against pressure, e.g. in the range as delivered, when tablets are pressed, thus allowing the use as active ingredient in preferred oral pharmaceutical formulation.

(rac)-N-piperidinyl-5-(4-chlorophenyl)-1-(2,4-dichlorophenyl)-4,5-dihydro-1 H-pyrazole-3-carboxamide and its preparation are described in detail in WO 2005/077911.

In another aspect of the present invention the $\alpha$-Polymorph of (rac)-N-piperidinyl-5-(4-chlorophenyl)-1-(2,4-dichlorophenyl)-4,5-dihydro-1H-pyrazole-3-carboxamide according to the invention crystallizes as a triclinic cell with an $\alpha$-angle of 78° and/or a $\beta$-angle of 74° and/or a $\gamma$-angle of 88°.

In another aspect of the present invention the $\alpha$-Polymorph of (rac)-N-piperidinyl-5-(4-chlorophenyl)-1-(2,4-dichlorophenyl)-4,5-dihydro-1H-pyrazole-3-carboxamide shows a standard X-Ray powder diffraction according to figure 8.

In another aspect of the present invention the $\alpha$-Polymorph of (rac)-N-piperidinyl-5-(4-chlorophenyl)-1-(2,4-dichlorophenyl)-4,5-dihydro-1H-pyrazole-3-carboxamide melts

above 180°C, preferably at an onset point of 183 $\pm$ 1 °C, more preferably at an onset point of 183-184 °C

and/or

with a melting peak at 185 $\pm$ 2 °C, preferably at 184 $\pm$ 1 °C.

In another aspect of the present invention the $\alpha$-Polymorph of (rac)-N-piperidinyl-5-(4-chlorophenyl)-1-(2,4-dichlorophenyl)-4,5-dihydro-1H-pyrazole-3-carboxamide decomposes at or above 280 °C.

In another aspect of the present invention the $\alpha$-Polymorph of (rac)-N-piperidinyl-5-(4-chlorophenyl)-1-(2,4-dichlorophenyl)-4,5-dihydro-1H-pyrazole-3-carboxamide shows in the FTRaman spectrum bands, preferably intense bands, at 2932 $\pm$ 5 $cm^{-1}$ and/or 1647 $\pm$ 5 $cm^{-1}$ and/or 1593 $\pm$ 5 $cm^{-1}$ and/or 1394 $\pm$ 5 $cm^{-1}$ and/or 1269 $\pm$ 5 $cm^{-1}$ and/or 1253 $\pm$ 5 $cm^{-1}$ and/or 1109 $\pm$ 5 $cm^{-1}$ and/or 1084 $\pm$ 5 $cm^{-1}$ and/or 1047 $\pm$ 5 $cm^{-1}$ and/or 783 $\pm$ 5 $cm^{-1}$ and/or 661 $\pm$ 5

$cm^{-1}$ and/or a Raman shift at 1253 ± 5 $cm^{-1}$ and/or at 1084 ± 5 $cm^{-1}$ and/or at 533 ± 5 $cm^{-1}$ and/or and at 364 $cm^{-1}$

It is highly preferred, if the α-Polymorph of (rac)-N-piperidinyl-5-(4-chlorophenyl)-1-(2,4-dichlorophenyl)-4,5-dihydro-1H-pyrazole-3-carboxamide shows at least 2, preferably at leat 3, more preferably at least 4, most preferably at least all 5 attributes from the following list:

- it crystallizes as a triclinic cell with an α-angle of 78° and/or a β-angle of 74° and/or a γ-angle of 88°;
- it shows a standard X-Ray powder diffraction according to figure 8;
- it melts
  above 180°C, preferably at an onset point of 183 ± 1 °C, more preferably at an onset point of 183-184 °C and/or
  with a melting peak at 185 ± 2 °C, preferably at 184 ± 1 °C;
- it decomposes at or above 280 °C;
- it shows in the FTRaman spectrum bands, preferably intense bands, at 2932 ± 5 $cm^{-1}$ and/or 1647 ± 5 $cm^{-1}$ and/or 1593 ± 5 $cm^{-1}$ and/or 1394 ± 5 $cm^{-1}$ and/or 1269 ± 5 $cm^{-1}$ and/or 1253 ± 5 $cm^{-1}$ and/or 1109 ± 5 $cm^{-1}$ and/or 1084 ± 5 $cm^{-1}$ and/or 1047 ± 5 $cm^{-1}$ and/or 783 ± 5 $cm^{-1}$ and/or 661 ± 5 $cm^{-1}$ and/or a Raman shift at 1253 ± 5 $cm^{-1}$ and/or at 1084 ± 5 $cm^{-1}$ and/or at 533 ± 5 $cm^{-1}$ and/or and at 364 $cm^{-1}$

In another aspect the present invention also provides a process for the manufacture of the α-Polymorph of (rac)-N-piperidinyl-5-(4-chlorophenyl)-1-(2,4-dichlorophenyl)-4,5-dihydro-1H-pyrazole-3-carboxamide according to the invention, wherein a solution of (rac)-N-piperidinyl-5-(4-chlorophenyl)-1-(2,4-dichlorophenyl)-4,5-dihydro-1H-pyrazole-3-carboxamide in an alcohol; preferably ethanol or methanol; is evaporated at room temperature (25 °C) to yield the α-Polymorph of (rac)-N-piperidinyl-5-(4-chlorophenyl)-1-(2,4-dichlorophenyl)-4,5-dihydro-1 H-pyrazole-3-carboxamide.

(rac)-N-piperidinyl-5-(4-chlorophenyl)-1-(2,4-dichlorophenyl)-4,5-dihydro-1H-pyrazole-3-carboxamide and its preparation are described in detail in WO 2005/077911. The respective descriptions are hereby incorporated by reference and form part of the present disclosure.

The Polymorph according to the invention, is suitable as pharmaceutical active substance for the preparation of medicaments.

It is found that (rac)-N-piperidinyl-5-(4-chlorophenyl)-1-(2,4-dichlorophenyl)-4,5-dihydro-1H-pyrazole-3-carboxamide and thus naturally its polymorph has a high affinity to cannabinoid receptors, particularly cannabinoid 1 ($CB_1$)-receptors, i.e. it is a selective ligand for the ($CB_1$)-receptor and acts as modulators, e.g. antagonists, inverse agonist or agonist, on these receptors. In particular, the compound shows little or no development of tolerance during treatment, particularly with respect to food intake, i.e. if the treatment is interrupted for a given period of time and then continued afterwards, the inventively used compound will again show the desired effect. After ending the treatment with the compound, the positive influence on the body weight is found to continue.

Furthermore, this compound shows relatively weak Herg channel affinity, thus a low risk of prolongation of the QT-interval is to be expected for this compound.

In summary, the compound is distinguished by a broad spectrum of beneficial effects, while at the same time showing relatively little undesired effects, i.e. effects which do not positively contribute to or even interfere with the well being of the patient.

Thus, another aspect of the present invention relates to a medicament comprising the α-Polymorph of (rac)-N-piperidinyl-5-(4-chlorophenyl)-1-(2,4-dichlorophenyl)-4,5-dihydro-1H-pyrazole-3-carboxamide according to the invention, and optionally one or more pharmaceutically acceptable excipients.

Another aspect of the present invention is the use of the α-Polymorph of (rac)-N-piperidinyl-5-(4-chlorophenyl)-1-(2,4-dichlorophenyl)-4,5-dihydro-1H-pyrazole-3-carboxamide according to the invention, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Also preferred is the use of the α-Polymorph of (rac)-N-piperidinyl-5-(4-chlorophenyl)-1-(2,4-dichlorophenyl)-4,5-dihydro-1 H-pyrazole-3-carboxamide according to the invention, and optionally one or more pharmaceutically acceptable excipients, for the prophylaxis and/or treatment of psychosis.

Also particularly preferred is the use of the α-Polymorph of (rac)-N-piperidinyl-5-(4-chlorophenyl)-1-(2,4-dichlorophenyl)-4,5-dihydro-1H-pyrazole-3-carboxamide according to the invention, and optionally one or more pharmaceutically acceptable excipients, for the preparation of a medicament for the prophylaxis and/or treatment of food intake

disorders, preferably bulimia, anorexia, cachexia, obesity and/or type II diabetus mellitus (non-insuline dependent diabetes mellitus), more preferably obesity.

Also preferred is the use of the α-Polymorph of (rac)-N-piperidinyl-5-(4-chlorophenyl)-1-(2,4-dichlorophenyl)-4,5-dihydro-1H-pyrazole-3-carboxamide according to the invention, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Also preferred is the use of the α-Polymorph of (rac)-N-piperidinyl-5-(4-chlorophenyl)-1-(2,4-dichlorophenyl)-4,5-dihydro-1H-pyrazole-3-carboxamide according to the invention, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencyclidine, hallucinogens and benzodiazepines.

Also preferred is the use of the α-Polymorph of (rac)-N-piperidinyl-5-(4-chlorophenyl)-1-(2,4-dichlorophenyl)-4,5-dihydro-1 H-pyrazole-3-carboxamide according to the invention, and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, , medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Also particularly preferred is the use of α-Polymorph of (rac)-N-piperidinyl-5-(4-chlorophenyl)-1-(2,4-dichlorophenyl)-4,5-dihydro-1H-pyrazole-3-carboxamide according to the invention, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof, and optionally one or more pharmaceutically acceptable excipients, for the preparation of a medicament for the prophylaxis and/or treatment of one or more disorders selected from the group consisting of dementia and related disorders, preferably for the prophylaxis and/or treatment of one or more types of dementia selected from the group consisting of memory loss, vascular dementia, mild cognitive impairment, frontotemporal dementia and Pick's disease; binge eating disorders; juvenile obesity; drug induced obesity; atypical depression; behavioural addictions; attention deficit disorders; Tourette's syndrome; suppression of reward-related behaviours; e. g. conditioned place avoidance such as suppression of cocaine- and morphine induced conditioned place preference; impulsivity; sexual dysfunction; preferably for the prophylaxis and/or treatment of one or more types of sexual dysfunction selected from the group consisting of erectile difficulty and female sexual dysfunction; seizure disorders; nausea; emesis; neuroinflammatory disease, preferably for the prophylaxis and/or treatment of one or more types of neuroinflammatory diseases selected from the group consisting of multiple sclerosis, demyelinisation related disorders, Guillan-Barré syndrome, viral encephalitis and cerebrovascular accidents; neurological disorders; muscle spasticity; traumatic brain injury; spinal cord injury; inflammation and immunomodulatory disorders, preferably for the treatment and/or prophylaxis of one or more types of inflammation and immunomodulatory disorders selected from the group consisting of cutaneous T-cell lymphoma, rheumatoid arthritis, systemic lupus erythematosus, sepsis, sarcoidosis, idiopathic pulmonary fibrosis, bronchopulmonary dysplasia, retinal disease, scleroderma, renal ischemia, mycocardial infarction, cerebral ischemia, nephritis, hepatitis, glomerulonephritis, cryptogenic fibrosing aveolitis, psoriasis, transplant rejection, atopic dermatitis, vasculitis, allergy, seasonal allergic rhinitis, Crohn's disease, inflammatory bowel disease, reversible airway obstruction, adult respiratory distress syndrome, chronic obstructive pulmonary disease and bronchitis; cerebral apoplexy; craniocerebral trauma; neuropathic pain disorders; gastric ulcers; atheriosclerosis and liver cirrhosis.

Dementia is a disease characterized by the progressive deterioration in cognitive and social adaptive functions that can eventually interfere with the patient's ability to live independently. Dementia also constitutes of impairment in short- and long-term memory plus additional symptoms, such as problems with abstract thinking, judgment, or personality. An estimated 18 million patients suffer from dementia worldwide. The most common forms of dementia include Alzheimer's disease and vascular dementia. Other forms are frontotemporal dementia and Pick's disease.

Dementia can also be of vascular origin. Vascular dementia (atherosclerotic cerebrovascular disease) is considered to be the second most common dementia of late life, affecting approximately 10-15% of all cases. Alzheimer's disease (AD) and vascular dementia can exist in isolation or together (mixed dementia). In vascular dementia, atherosclerotic changes in cerebral vessels can lead to reduced local blood flow that results in multiple small strokes (multi-infarct dementia). Vascular dementia is pharmacologically treated by stroke prophylaxis, and by treatment of the cognitive deficit.

Alzheimer's disease (AD), the most common and important form of dementia, is a neurodegenerative disorder that is characterized by progressive impairment of cognitive functions, such as abstract reasoning and memory. Currently, an estimated 2 million people in the United States and 12 million worldwide are afflicted by this disease. Due to increasing life expectancy, it is predicted that there will be over 100 million AD patients worldwide by the year 2050. AD is one of the most prevalent illnesses in the elderly. The majority of AD patients are in their sixties or older. More than 5% of all persons over the age of 70 have significant memory loss due to AD.

AD is mainly characterized through a gradual development of forgetfulness. In further advanced disease stages, other failures in cerebral function become increasingly apparent. This includes impairment of speech, writing, and arithmetic skills. Visiospacial orientation, such as parking the car, dressing properly, and giving and understanding directions to a location, can become defective or impaired. In late stage disease, patients forget how to use common objects and tools while retaining necessary motor power and co-ordination for these activities.

Schizophrenia is characterized by profound disruption in cognition and emotion, affecting the most fundamental human attributes: language, thought, perception, affect, and sense of self. Positive symptoms include psychotic manifestations, such as hearing internal voices or experiencing other sensations not connected to an obvious source (hallucinations) and assigning unusual significance or meaning to normal events or holding fixed false personal beliefs (delusions). Negative symptoms are characterized by affective flattening and lack of initiative or goals (avolition), loss of usual interests or pleasures (anhedonia), disturbances of sleep and eating, dysphoric mood (depressed, anxious, irritable, or angry mood) and difficulty concentrating or focusing attention.

Major depression is a multifaceted disorder characterized by primarily by dysphoric mood and loss of interest or pleasure in activities that were once enjoyable. Other physical and psychological symptoms include inability to concentrate, motor disturbances (psychomotor retardation or agitation), feelings of worthlessness, inappropriate guilt, thoughts of suicide, and disturbances in appetite and sleep.

Anxiety disorders are a group of syndromes that include generalized anxiety disorder, panic disorder, phobias, obsessive-compulsive disorder, and post traumatic stress disorder. Although each disorder has its own distinct features, all share common symptoms of excessive worrying, intense fears and dread, hypervigilance and/or somatic symptoms, in the absence of a dangerous situation.

Normal sexual function requires, among others, the ability to achieve and maintain penile erection. Major anatomic structures of the penis that are involved in erectile function include the corpus cavernosum, corpus spinosum, and the tunica albuginea (a collagenous sheath that surrounds each corpus). The corpora are composed of a mass of smooth muscle (trabecula) which contains a network of endothelial-lined vessels (lacunar spaces). Penile tumescence and erection is caused by relaxation of the arteries and corporal smooth muscles, while closing emissary veins, leading to increased blood flow into the lacunar network. Central and peripheral innervation contributes to regulation of the erectile response.

Erectile dysfunction (ED) may result from failure to initiate, fill, or store adequate blood volume within the lacunar network of the penis. Depending on the underlying dysfunction, ED may be vasculogenic, neurogenic, endocrinologic, diabetic, psychogenic, or medication-related.

ED affects 10-25% of middle-aged and elderly men, and has a profound impact on the well-being of affected men. It is currently treated using PDE5 inhibitors such as vardenafil, tadalifil, and sildenafil. Intraurethral alpostadil (prostaglandin EI) may be used in patients that fail on oral agents. In addition, vacuum constriction devices (VCD) are a well-established, noninvasive therapy.

Female sexual dysfunction (FSD) is highly prevalent, age-related, and progressive. It affects 30 to 50% of women. FSD denotes a range of medical problems and is categorized according to disorders of (1) desire, (2) arousal, (3) orgasm and (4) sexual pain, and symptoms include diminished vaginal lubrication, pain and discomfort with intercourse, decreased arousal, and difficulty achieveing orgasm. On a molecular level, vasoactive intestinal peptide (VIP), nitic oxide (NO), and sex hormones such as estrogens and androgens have been suggested to be important in female sexual function. Current treatment approaches include estrogen replacement therapy, methyl testosterone, PDE5 inhibitors such as sildenafil, the NO-donor L-arginine, prostaglandin EI, phentolamine, and the dopamine agonists apomorphine.

Also preferred is the use of the α-Polymorph of (rac)-N-piperidinyl-5-(4-chlorophenyl)-1-(2,4-dichlorophenyl)-4,5-dihydro-1 H-pyrazole-3-carboxamide according to the invention, and optionally one or more pharmaceutically acceptable excipients, for the preparation of a medicament for the prophylaxis and/or treatment of metabolic syndrome,

especially weight independent, cardiovascular diseases, cardiovascular risk factors, glucose intolerance and insulin resistance; for the prophylaxis and/or treatment and/or influencing of dyslipidaemia; for the prophylaxis and/or treatment and/or influencing of blood parameters especially lipid parameters, seeming to lower LDL bodies while increasing HDL bodies, including also the treatment of food disorders (obesity) under conditions of developed diabetes, especially Type II.

The metabolic syndrome and definitions thereof are described in detail by Eckel et al., The Lancet, Vol. 365 (2005), 1415-1428, included herewith by reference. One of the respective definitions was established by the WHO in 1998 (as described in Alberti et al., Diabet. Med. 1998, 15, pages 539-53, the respective description thereof is herewith incorporated by reference and forms part of the present disclosure). The other, more widely accepted, definition of the metabolic syndrome was established by the Adult Treatment Panel (ATP III) of the US National Cholesterol Education Program (NCEP) in 2001, as described in JAMA 2001; 285; 2486-97, the respective description thereof is herewith incorporated by reference and forms part of the present disclosure.

The metabolic syndrome is characterized by an interaction of several physiological parameters such as triglycerides, lipids, blood pressure, glucose levels and insuline levels. Even though obesity may play a critical role in the development of metabolic syndrome, many of its aspects are weight independent, especially some lipid parameters. Especially the positive influence on the weight independent aspects of the metabolic syndrome (see e.g. Pagotto and Pasquali, The Lancet, Vol. 365 (2005), 1363, 1364, included herewith by reference) like some blood parameters, especially lipid parameters is one of the major and surprising advantages of the inventively used substituted pyrazoline compounds.

Since the α-Polymorph of (rac)-N-piperidinyl-5-(4-chlorophenyl)-1-(2,4-dichlorophenyl)-4,5-dihydro-1H-pyrazole-3-carboxamide according to the invention also has a beneficial effect on the ratio of low density lipoprotein (LDL) to high density lipoprotein (HDL), i.e. they lower the LDL-levels and/or elevate the HDL levels; they are also useful as coating material or co-coating material in stents in order to prevent restenosis.

The term "treatment" as used in the present application encompasses prevention, amelioration and/or complete recovery from the disease. Said term also includes the prevention, amelioration and/or complete recovery of one or more symptoms associated with the disease.

Furthermore, the α-Polymorph of (rac)-N-piperidinyl-5-(4-chlorophenyl)-1-(2,4-dichlorophenyl)-4,5-dihydro-1 H-pyrazole-3-carboxamide according to the invention is connected to relatively weak Herg channel affinity, thus a low risk of prolongation of the QT-interval is to be expected for the compound.

In summary, the inventively used α-Polymorph of (rac)-N-piperidinyl-5-(4-chlorophenyl)-1-(2,4-dichlorophenyl)-4,5-dihydro-1 H-pyrazole-3-carboxamide according to the invention is distinguished by a broad spectrum of beneficial effects, while at the same time showing relatively little undesired effects, i.e. effects which do not positively contribute to or even interfere with the well being of the patient.

Another aspect of the invention is the use of the α-Polymorph of (rac)-N-piperidinyl-5-(4-chlorophenyl)-1-(2,4-dichlorophenyl)-4,5-dihydro-1H-pyrazole-3-carboxamide according to the invention for the manufacture of a medicament for improvement of cardiovascular and/or metabolic risk factors, such as one or more of the following factors:

- Elevated triglycerides, whereby elevated levels of triglycerides are preferably understood as being > 150 mg/dl,
- Low HDL cholesterol, whereby low levels of HDL cholesterol are preferably understood as being < 40 mg/dl in men and < 50 mg/dl in women,
- Hypertension, whereby hypertension is preferably understood as being > 130/85 mmHg,
- Impaired fasting glucose, whereby impaired fasting glucose levels are preferably understood as being > 110 mg/dl,
- Insulin resistance
- Dyslipidemia.

Another aspect of the invention is the use of the α-Polymorph of (rac)-N-piperidinyl-5-(4-chlorophenyl)-1-(2,4-dichlorophenyl)-4,5-dihydro-1 H-pyrazole-3-carboxamide according to the invention for the manufacture of a medicament for the treatment of the weight independent aspects of metabolic syndrome.

Another very preferred aspect of the invention is also the use of the α-Polymorph of (rac)-N-piperidinyl-5-(4-chlorophenyl)-1-(2,4-dichlorophenyl)-4,5-dihydro-1 H-pyrazole-3-carboxamide according to the invention for the manufacture of a medicament for the treatment of dyslipidemia.

Another very preferred aspect of the invention is also the use of the α-Polymorph of (rac)-N-piperidinyl-5-(4-chlorophenyl)-1-(2,4-dichlorophenyl)-4,5-dihydro-1H-pyrazole-3-carboxamide according to the invention for the manufacture of a medicament for the treatment of dyslipidemia or metabolic syndrome.

Another aspect of the invention is a method for improving cardiovascular and/or metabolic risk factors, such as one or more of the following factors:

- Elevated triglycerides, whereby elevated levels of triglycerides are preferably understood as being > 150 mg/dl,
- Low HDL cholesterol, whereby low levels of HDL cholesterol are preferably understood as being < 40 mg/dl in men and < 50 mg/dl in women,

- Hypertension, whereby hypertension is preferably understood as being > 130/85 mmHg,
- Impaired fasting glucose, whereby impaired fasting glucose levels are preferably understood as being > 110 mg/dl,
- Insulin resistance
- Dyslipidemia,

in a subject, preferably a human.

Another preferred aspect of the invention is also a method of treatment encompassing all the abovementioned uses, wherein the α-Polymorph of (rac)-N-piperidinyl-5-(4-chlorophenyl)-1-(2,4-dichlorophenyl)-4,5-dihydro-1H-pyrazole-3-carboxamide according to the invention, is applied to a person in need thereof, treating metabolic syndrome, especially weight independent, cardiovascular diseases especially fighting cardiovascular risk factors, influencing the blood parameters, especially the lipid parameters, diabetes, especially type II, glucose intolerance and insulin resistance, bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit; alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer; food intake disorders, preferably bulimia, anorexia, cachexia, obesity and/or type II diabetus mellitus (non-insuline dependent diabetes mellitus), more preferably obesity; psychosis; disorders of the central nervous system, disorders of the immune system, disorders of the cardiovascular system, disorders of the endocrinous system, disorders of the respiratory system, disorders of the gastrointestinal tract or reproductive disorders.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams or also 20 to 200 mg of active substance to be administered during one or several intakes per day.

**Pharmacological Methods:**

**I. In-vitro determination of affinity to CB1/CB2-Receptors**

a)

The in-vitro determination of the affinity of the inventive substituted pyrazoline compounds to $CB_1$/$CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is [$^3$H]-CP55940. The respective parts of the description is hereby incorporated by reference and forms part of the present disclosure.

b)

Rat cerebellum CB1 binding

Binding affinity to CB1 receptor was evaluated according to a modification of the method described by Govaerts et al., Eur J Pharmac Sci 23, 233-243 (2004). The respective parts of the description is hereby incorporated by reference and forms part of the present disclosure.

Briefly, cerebellum from male wistar rats (250-300g) were carefully dissected on ice and homogenates were prepared with a Potter-Helveheim homogeniser a cold 50 mM Tris-HCl solution containing 5 mM $MgCl_2$, 1 mM EDTA and 0.25 M sucrose, pH 7.4. The suspension was centrifuged at 1,000 x g for 5 minutes. The supernatants were collected and centrifuged 50,000 x g for 15 minutes. The resulting pellets were then resuspended in Tris-HCl buffer without sucrose, homogenized and incubated for 15 min at 37°C in an orbital shaker bath and centrifuged again at 50,000 x g for 15 min. Pellets were weighted, resuspended in Tris-HCl buffer without sucrose, homogenized with an Ultraturrax homogeniser at 13,500 rpm for 3 x 5 seconds and aliquoted in 0.9 ml volumes in Eppendorf tubes. Alicuotes were centrifuged at 20,800 x g for 5 minutes, supernatants discarded and pellets were frozen at - 80°C until use. Total protein concentration was determined using the Bio-Rad commercial assay based on the Lowry method.

Competitive binding experiments were performed in presence of 1 nM [$^3$H]-CP 55,940 in siliconized glass tubes containing 100 μg protein/tube resuspended in 1 ml final volume of 50 mM Tris-HCl, 5 mM $MgCl_2$, 1 mM EDTA, 0.5% (w/v) bovine serum albumin, pH 7.4. Compounds were present at various concentrations and the non specific binding was determined in the presence of 10 μM HU-210. After 1 hour incubation at 30°C, the suspension was rapidly filtered through 0.5% PEI pre-treated GF/B fiber filters on a 96-well harvester and washed 3 times with 3 ml ice-cold binding buffer without bovine serum albumin. Radioactivity on filters was measured with Wallac Winspectral 1414 counter by liquid scintillation in 6 ml Ecoscint H (National Diagnostics, U.K.). Assays were made in triplicates.

Binding data were analyzed by non-linear regression with the software GraphPad Prism Version 3.03.

**II. In-vivo bioassay system for determination of cannabinoid activity**

**Mouse tetrad model**

Substances with affinity for cannabinoid receptors are known to produce a wide range of pharmacological effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are highly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54, 161-202 , 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

## Material and Methods

Male NMRI mice with a weight of 20-30 g (Harlan, Barcelona, Spain) are used in all of the following experiments.

Before testing in the behavioural procedures given below, mice are acclimatised to the experimental setting. Pre-treatment control values are determined for analgesia hot plate latency (in seconds), rectal temperature, sedation and catalepsy.

In order to determine the agonistic activity of the substance to be tested, the mice are injected intravenously with the substance to be tested or the vehicle alone. 15 minutes after injection, latency in hot plate analgesia is measured. Rectal temperature, sedation and catalepsy are measured 20 minutes after injection.

In order to determine the antagonistic activity the identical procedure is used as for the determination of the agonistic effects, but with the difference that the substance to be evaluated for its antagonistic activity is injected 5 minutes before the intravenous injection of 1.25 mg/kg Win-55,212 a known cannabinoid-receptor agonist.

## Hot plate analgesia

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.

The mice are placed on a hot plate (Harvard Analgesimeter) at 55 ± 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).

Fifteen minutes after the administration of the substance to be tested, the mice are again placed on the hot plate and the afore described procedure is repeated. This period is called the post-treatment reading (PT).

The degree of analgesia is calculated from the formula :

$$\%\ \text{MPE of Analgesia} = (\text{PT} - \text{B}) / (\text{PC} - \text{B}) \times 100$$

MPE = Maximum possible effect.

## Determination of sedation and ataxia

Sedation and ataxia is determined according to the method described in Desmet L. K. C. et al. "Anticonvulsive properties of Cinarizine and Flunarizine in Rats and Mice", Arzneim. - Forsch. (Frug Res) 25, 9, 1975. The respective description is hereby incorporated by reference and forms part of the present disclosure.

The chosen scoring system is
**0**: no ataxia;
**1**: doubtful;
**2**: obvious calmness and quiet;
**3** pronounced ataxia;
prior to as well as after treatment.

The percentage of sedation is determined according to the formula:

$$\%\ \text{of sedation} = \text{arithmetic mean} / 3 \times 100$$

## Hypothermia:

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.

The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is

calculated for each animal, whereby differences of ≥ -2 °C are considered to represent activity.

**Catalepsy:**

Catalepsy is determined according to the method described in Alpermann H. G. et al. "Pharmacological effects of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for:

more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according to the following formula:

$$\% \text{ Catalepsy} = \text{arithmetic mean} / 6 \times 100$$

**III. In vivo testing for antiobesic activity**

a) Accute Treatment

Normally handled rats are habituated to a reversed cycle 12/12h, and the tested compound as well as saline was acutely orally administered. After administration the cumulated food intake (g) is measured at 6 h and 24 h. Following that the difference in body weight between control and compound treated animals is measured. This is a variation of the test according to Colombo et al. as described below.

b) Long-term Treatment

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

**IV. In vivo testing for antidepressant activity**

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.

**V. Determination of the effect of continued administration of the inventive compounds on body weight in rats**

The weight of the animals was measured for 36 days, and the data grouped into 3 periods for analysis: Period 1, days 1-8 (period for adaptation of the animals to the environmental conditions: one week without treatment plus first day of treatment); Period 2, days 9-22 (treatment period: two weeks); Period 3, days 23-36 (escape treatment period: two weeks). The administration of the drugs started on day 8, immediately after the animal was weighted, and finished on day 21. The treatment period ranges between one day after first administration and one day after last administration. The drugs were administered i.p. once daily. For comparative purposes, absolute weights were transformed into relative percentages (each weight was divided by that of day 8 and multiplied by 100). Three treatment groups were constructed: vehicle, racemic N-piperidinyl-5-(4-chlorophenyl)-1-(2,4-dichlorophenyl)-4,5-dihydro-1 H-pyrazole-3-carboxamide (10 mg/kg), and (rac)-N-piperidinyl-5-(4-chlorophenyl)-1-(2,4-dichlorophenyl)-4,5-dihydro-1 H-pyrazole-3-carboxamide (10

mg/kg).

### *VI. In vitro determination* of *antagonism* to *CB1-receptor*

**Membrane preparation:**

Chinese hamster ovary (CHO) cells stably expressing recombinant human cannabinoid 1 receptor (CB1) are cultured in nutrient mixture Ham's F 12 supplemented with 10 % heat-inactivated fetal bovine serum, 2 mM L-glutamine, 50 U/ml penicillin, 50 U/ml streptomycin and 0.5 mg/ml geneticin. In order to obtain cells, culture flasks are washed twice with phosphate buffered saline and scraped. Then, cells are collected by centrifugation (200 x g, 10 min) and stored dry at -80°C. Cells are homogenized in ice-cold 20 mM HEPES, 10 mM EDTA (pH 7.5) and centrifuged at 40,000 x g for 15 min at 4°C. The membrane pellet is resuspended in 20 mM HEPES, 0.1 mM EDTA (pH 7.5) and centrifuged for 15 min at 4°C. The final membrane pellet is resuspended in 20 mM HEPES, 0.1 mM EDTA (pH 7.5), and divided in aliquots and stored at -80°C until use.

**[$^{35}$S]GTPγS binding assay:**

The reaction is performed in 96-well plates. Membranes (15 µg protein/well) are incubated for 60 min at 30 °C in buffer (50 mM HEPES, 100 mM KCI, 5 mM $MgCl_2$,1 mM EDTA, 0.1 % wt/vol bovine serum albumin, 5 µM GDP, saponin (10 µg/ml), 0.5 nM [$^{35}$S]GTPγS, pH 7.4) with compound at a final concentration of 1 µM in either the absence or presence of agonist WIN 55,212-2 between 3 nM and 3 µM. The incubation is terminated by rapid filtration through Millipore Multiscreen glass fiber FB, and rinsed two-times with ice-cold assay buffer. Filter plates are dried and 30 µl of scintillation liquid is added. Radioactivity is determined using a Wallac Microbeta Trilux. Each experiment is performed at least in duplicate. A WIN 55,212-2 dose-response experiment either alone or in the presence of Rimonabant (1 µM) is systematically performed.

**Calculations:**

The average of basal [$^{35}$S]GTPγS binding is subtracted from all binding data. In order to compare the antagonism results from one screening campaign to another one, the difference between the maximal agonist effect of WIN 55,212-2 alone, and the maximal antagonism effect due to WIN 55,212-2 plus Rimonabant (1 µM) is defined as 100 %.

**Further methods:**

**Alcohol Intake**

The following protocol may be used to evaluate the effects of alcohol intake in alcohol preferring (P) female rats (e.g. bred at Indiana University) with an extensive drinking history. The following reference provides detailed a description of P rats: Lumeng, L, et al.,"Different sensitivities to ethanol in alcohol-preferring and-nonpreferring rats,"Pharmacol, Biochem Behav., 16, 125-130 (1982).

Female rats are given 2 hours of access to alcohol (10% v/v and water, 2-bottle choice) daily at the onset of the dark cycle. The rats are maintained on a reverse cycle to facilitate experimenter interactions. The animals are initially assigned to four groups equated for alcohol intakes: Group 1-vehicle; Group 2-positive control (e. g. 5.6 mg/kg AM251; Group3-low dose test compound; and Group 4-high dose of test compound. Test compounds are generally mixed into a vehicle of 30% (w/v) -cyclodextrin in distilled water at a volume of 1-2 ml/kg. Vehicle injections are given to all groups for the first two days of the experiment. This is followed by 2 days of drug injections (to the appropriate groups) and a final day of vehicle injections. On the drug injection days, drugs are given sc 30 minutes prior to a 2-hour alcohol access period. Alcohol intake for all animals is measured during the test period and a comparison is made between drug and vehicle-treated animals to determine effects of the compounds on alcohol drinking behavior.

Additional drinking studies can be done utilizing female C57Bl/6 mice (Charles River). Several studies have shown that this strain of mice will readily consume alcohol with little to no manipulation required (Middaugh et al.,"Ethanol Consumption by C57BU6 Mice : Influence of Gender and Procedural Variables" Alcohol, 17 (3),175-183, 1999; Le et al.,"Alcohol Consumption by C57BL/6, BALA/c, and DBA/2 Mice in a Limited AccessParadigm" PharmacologyBiochemistry and Behavior,47, 375-378,1994).

For example, upon arrival mice are individually housed and given unlimited access to powdered rat chow, water and a 10 % (w/v) alcohol solution. After 2-3 weeks of unlimited access, water is restricted for 20 hours and alcohol is restricted to only 2 hours access daily. This is done in a manner that the access period was the last 2 hours of the dark part of the light cycle.

Once drinking behavior is stabilized, testing can commence. Mice are considered stable when the average alcohol consumption for 3 days is 20% of the average for all 3 days. Day 1 of test consists of all mice receiving vehicle injection (sc or ip). Thirty to 120 minutes post injection access is given to alcohol and water. Alcohol consumption for that day is calculated (g/kg) and groups are assigned so that all groups have equivocal alcohol intake. On day 2 and 3, mice are injected with vehicle or drug and the same protocol as the previous day is followed. Day 4 is wash out and no injections are given. Data is analyzed using repeated measures ANOVA. Change in water or alcohol consumption is compared back to vehicle for each day of the test. Positive results would be interpreted as a compound that was able to significantly reduce alcohol consumption while having no effect on water

**Oxygen Consumption Methods:**

Whole body oxygen consumption is measured using an indirect calorimeter (Oxymax from Columbus Instruments, Columbus, OH) in male Sprague Dawley rats (if another rat strain or female rats are used, it will be specified). Rats (e.g. 300-380 g body weight) are placed in the calorimeter chambers and the chambers are placed in activity monitors. These studies are done during the light cycle. Prior to the measurement of oxygen consumption, the rats are fed standard chow ad libitum. During the measurement of oxygen consumption, food is not available. Basal pre-dose oxygen consumption and ambulatory activity are measured every 10 minutes for 2.5 to 3 hours. At the end of the basal pre-dosing period, the chambers are opened and the animals are administered a single dose of compound (the usual dose range is 0.001 to 10 mg/kg) by oral gavage (or other route of administration as specified, i. e. , sc, ip, iv). Drugs are prepared in methylcellulose, water or other specified vehicle (examples include PEG400, 30% beta-cyclo dextran and propylene glycol). Oxygen consumption and ambulatory activity are measured every 10 minutes for an additional 1-6 hours post-dosing.

The Oxymax calorimeter software calculates the oxygen consumption (ml/kg/h) based on the flow rate of air through the chambers and difference in oxygen content at inlet and output ports. The activity monitors have 15 infrared light beams spaced one inch apart on each axis, ambulatory activity is recorded when two consecutive beams are broken and the results are recorded as counts.

Resting oxygen consumption, during pre-and post-dosing, is calculated by averaging the 10-min02 consumption values, excluding periods of high ambulatory activity (ambulatory activity count > 100) and excluding the first 5 values of the pre-dose period and the first value from the post-dose period. Change in oxygen consumption is reported as percent and is calculated by dividing the post-dosing resting oxygen consumption by the pre-dose oxygen consumption *100. Experiments will typically be done with n = 4-6 rats and results reported are mean +/-SEM.

Interpretation :

An increase in oxygen consumption of > 10% is considered a positive result. Historically, vehicle-treated rats have no change in oxygen consumption from pre-dose basal.

**Nicotine Dependence**

An intravenous nicotine self-administration model or place preference model may be used to assess the effects of a test compound on nicotine dependence (see, e.g., Vastola, et al. Physiol. Behav. 77:107-114, 2002; Brower, et al., Brain Res. 930:12-20, 2002).

**Place Preference**

Sprague-Dawley rats are used in this study (Vastola, et al., 2002). Animals are housed in a temperature-controlled, 12h/12h illumination cycle with ad libitum access to food and water. Conditioning and testing are conducted in a chamber divided into two compartments with a door separating the two compartments. Behavior of the animals is recorded by video camera.

Animals are habituated to the injection procedure for several days. The animals are then placed into the test chamber and given free access to both compartments. The initial preference for a particular compartment is determined. For the conditioning trials, animals are injected with nicotine and restricted to the nonpreferred compartment, or the animals are injected with saline and restricted to the preferred compartment. On test day, the door separating the compartments is removed, the animal is placed in the center of the chamber and allowed to move freely between compartments. Time spent in each compartment is scored. Preferential occupancy of the nicotine compartment follows from the conditioned reinforcing effects of nicotine.

## Self-administration

Self-administration in animals is a predictor of a compound's abuse potential in humans. Modifications to this procedure may also be used to identify compounds that prevent or block the reinforcing properties of drugs that have abuse potential. A compound that extinguishes the self- administration of a drug may prevent that drug's abuse or its dependence.

Sprague-Dawley rats are used in this study. Initially, animals are housed in a temperature-controlled, 12h/12h illumination cycle with ad libitum access to food and water. The animals are then implanted with jugular catheters which exit through the animal's back, and each animal is placed in an individual operant chamber (Brower, et al., 2002). The catheters are connected to a computer-driven syringe pump which is located outside of the chamber. The chamber contains two levers with a green light located above each lever. The light is illuminated when nicotine is available.

In a self-administration test, animals are placed in the operant chambers and the levers are randomly designated as an active and inactive lever. Each response on the active lever produces an infusion of nicotine. Presses on the inactive lever have no effect, but are also recorded. Animals are then trained to self -administer nicotine over a set period of time by having drug access during each daily session. Illumination of the chamber house light signals the beginning of the session and the availability of nicotine. When the session ends, the house light is turned off. Initially, a nicotine infusion occurs with every press of the active lever. Once lever-pressing behavior has been established, the number of presses to produce a nicotine infusion is increased. After stable nicotine self-administration is obtained, the effect of a test compound on the nicotine- reinforced behavior may be evaluated. Administration of this test compound prior to the session can either potentiate, extinguish, or produce no change to the self-administrating behavior. Tests are conducted every two days, and the order of the administration of the test compound doses is controlled.

## ALZHEIMER/DEMENTIA EXPERIMENTS

## Morris Water Maze Task

The Morris water maze is a behavioral in vivo test to measure spatial orientation learning and memory through a complex learning task. It is highly suitable for testing compounds that enhance learning and memory. A circular water tank or pool (diameter 2 m, height 0.7 m) is filled with water, and a 10 $cm^2$ platform is placed 1-1.5 cm below the water surface at a defined location within the pool. The escape platform is not visible for an animal swimming in the water tank. For the experiment, a rat or mouse is placed into the pool to swim freely.

The animals have the task to localize the submerged platform, and the time and distance required for successful retrieval is measured. Multiple extra-maze cues are provided by the furniture in the room, including desks, computer equipment, a second water tank, the presence of the experimenter, and by a radio on a shelf that is playing softly.

Before administration of the test compound, animals are usually trained in the task 4 times a day for 5 days. Test compounds are administered orally or intraperitoneally on the day of the experiment at a defined time (e.g., 30 minutes before the first swim test). Control animals are dosed with the corresponding vehicle not containing test compound. Active compounds yield shorter times and distances to localize the platform (i.e., the better the animal remembers the location of the platform, the shorter the distance covered and the faster the platform is reached).

The test can also be carried out using transgenic or cognitively impaired animals. Cognitive impairment is induced either by old age or experimentally through brain lesions, such as bilateral lesions of the entorhinal cortex in rats. Such lesions can be induced by intracerebral injections of the excitotoxin ibotenic acid.

## Object Recognition Task

The object recognition task is used to assess the effects of compounds on the cognitive performance of rodents. A rat is placed in an open field, in which two identical objects are located. The rats inspects both objects during the initial trial of the test. After a certain retention interval (e.g., 24 hours), a second trial is carried out. Here, one of the two objects used in the first trial (the 'familiar' object) and a novel object are placed in the open field, and the inspection time at each of the objects is measured. Good retention is reflected by higher exploration times towards the novel compared with the 'familiar' object.

Administration of the putative cognition enhancer prior to the first trial predominantly allows assessment of the effects on acquisition, and on the consolidation processes. Administration of the test compound after the first trial allows to assess the effects on consolidation processes, whereas administration before the second trial allows to measure effects on retrieval processes.

## Passive Avoidance Task

The passive avoidance task assesses memory performance in rats and mice. The inhibitory avoidance uses an apparatus consisting of a box with two compartments separated by a guillotine door that can be operated by the experimenter. One compartment is illuminated with bright light, and the other compartment is dark. A threshold of 2 cm separates the two compartments when the guillotine door is raised. When the door is open, the illumination in the dark compartment is about 2 lux. The light intensity is about 500 lux at the center of the floor of the light compartment.

Two habituation sessions, one shock session, and a retention session are given, separated by inter-session intervals of 24 hours. During the habituation sessions and the retention session, the rat is allowed to explore the apparatus for 300 seconds. The rat is placed in the light compartment, facing the wall opposite to the guillotine door. After an accommodation period of 15 seconds, the guillotine door is opened so that all parts of the apparatus can be visited freely. Rats normally avoid brightly lit areas and will enter the dark compartment within a few seconds.

In the shock session, the guillotine door between the compartments is lowered as soon as the rat has entered the dark compartment with all paws, and a scrambled 1 mA footshock is administered for 2 seconds. Then the rat is removed from the apparatus and returned into its home cage. The procedure during the retention session is identical to that of the habituation sessions.

The step-through latency, that is, the first latency of entering the dark compartment (in seconds) during the retention session is an index of the memory performance of the animal: a better retention is assumed if the latency to enter the dark compartment is longer. A test compound is given 30 minutes before the shock session, together with 1 mg/kg scopolamine. Scopolamine impairs the memory performance during the retention session 24 hours later. If the test compound increases the enter latency compared with the scopolamine-treated controls, it is considered to possess cognition enhancing activity. T-maze Spontaneous Alternation Task

The T-maze spontaneous alternation task (TeMCAT) assesses the spatial memory performance in mice. The start arm and the two goal arms of the T-maze are provided with guillotine doors that can be operated manually by the experimenter. A mouse is put into the start arm at the beginning of training. In the first trial, either the left or right goal arm is blocked by lowering the respective guillotine door (forced trial).

After the mouse has been released from the start arm, it will explore the maze, eventually entering the open goal arm, and return to the start position, where it will be confined for 5 seconds, by lowering the guillotine door. Then, the animal can choose freely between the left and right goal arm (all guillotine-doors opened) during 14 additional trials (free choice trials). As soon as a mouse has entered one goal arm, the other arm is closed. The mouse eventually returns to the start arm and is free to visit whichever arm it wants after having been confined to the start arm for 5 seconds. After completion of 14 free choice trials in one session, the animal is removed from the maze.

Out of the 14 trials the alternations in percent are calculated. This percentage and the total time needed to complete the first forced trial and the subsequent 14 free choice trials (in seconds) is analyzed. In addition, cognitive deficits can be induced by injection of scopolamine 30 minutes before the start of the training session. A cognition enhancer, administered before the training session, will at least partially, antagonize the scopolamine-induced reduction in the spontaneous alternation rate.

## Depression Model

A forced swim or tail suspension model may be used to assess the efficacy of antidepressant compounds (see , e.g., Porsolt, et al., Nature 266:730-732, 1977; Stem, et al., Psychopharmacology 85:367-370, 1985).

## Forced Swim Test

Rats or mice are placed in a cylinder filled with water 23-25 °C from which no escape is possible. Initially, animals struggle and try to escape, but eventually adopt a characteristic immobile posture and make no further attempts to escape except for small movements needed their head above water. Animals are dosed with a compound and the activity (swimming or climbing) or immobility is measured by an observer. The immobility is considered by some to reflect a 'behavioral despair' in which animals cease to struggle to escape the aversive situation. A wide variety of clinically used antidepressants (TCAs, MAOIs, SSRIs, atypicals) decrease immobility in this test and has a good predictive validity in that it detects antidepressants with different mechanisms of action but its construct validity is weak. At least two distinct active behavioral patterns are produced by pharmacologically selective antidepressant drugs. Serotonin-selective reuptake inhibitors increase swimming behavior, whereas drugs acting primarily to increase extracellular levels of norepinephrine or dopamine increase climbing behavior. There are false positives (psychostimulants) but relatively few false negatives ([beta]-adrenergic agonists). The test is sensitive to muscle-relaxant (benzodiazepines) and sedative (neuroleptics) effects, leading to enhanced immobility. False positives and false negatives can often be screened by measuring if the compound produces locomotor stimulation or sedation.

**Tail Suspension Test**

When suspended by the tail, mice will initially struggle and try to escape and then alternate between active escape attempts and immobililty. In this test, animals are dosed with a compound and the immobility is measured by an observer for 6 min. Porsolt describes the immobile behavior as 'behavioral despair' which animals cease to struggle to escape the aversive situation. A large variety of clinically antidepressants (tricyclics, MAOIs, SSRIs, and atypicals) reduce immobility in this model. The test has a good predictive validity for antidepressant activity and works for most antidepressant classes including but has some false positives (psychostimulants). The test is sensitive to muscle-relaxant (benzodiazepines) and sedative (neuroleptics) effects, which lead to enhanced immobility. False positives and false negatives can often be screened by measuring if the compound produces locomotor stimulation or sedation. Strain differences in the tail suspension test have been found in mice. The tail suspension test has some face validity but its construct validity is rather weak.

**Schizophrenia Model**

A prepulse inhibition model may be used to assess the efficacy of antipsychotic compounds (see Swerdlow and Geyer, Schizophrenia Bulletin 24: 285-301, 1998).

**Prepulse Inhibition**

Prepulse inhibition is the process whereby a relatively mild stimulus, the prepulse, suppresses the response to a strong, startle-eliciting stimulus when the prepulse precedes the startle stimulus by a brief duration (about 10 to 500 milliseconds). Prepulse inhibition is a cross-species phenomenon (ie, it is present in mammals ranging from mice to humans), yet it is relatively absent among schizophrenic patients. The deficit in PPI in schizophrenic patients is thought to reflect the loss of sensorimotor gating that may lead to sensory flooding and cognitive fragmentation. In this test, mice or rats are administered compounds and individually placed into a holder on a transducer platform to measure whole body startle. The holder is housed in a startle chamber with background white noise. Following a brief habituation period, animals are given multiple trials of a weak auditory prepulse stimululs, followed by a strong auditory startle stimulus. Four types of trials are given: prepulse plus startle, prepulse alone, startle alone, and no stimulation. PPI is measured as the amount of inhibition of startle following the prepulse and is expressed as the percentage of basic startle. As a control, measurements are taken in the no stimulation and prepulse alone trials. PPI is considered a test with good predictive, face and construct validity for schizophrenia. Putative antipsychotics can be tested alone to determine if they enhance PPI. Alternately, antipsychotics can be screened to determine if they block various agents that disrupt PPI (apomorphine, d- amphetamine, PCP, ketamine, DOI). Finally, mutant mice with or without drugs can be screened using the PPI procedure.

**Anxiety Model**

An elevated plus maze model may be used to assess the efficacy of anxiolytic compounds (see Pellow and File, Pharm. Biochem. Behav. 24, 525-529, 1986).

**Elevated Plus Maze**

The elevated plus maze is widely used as an anxiety paradigm that examines the conflict between the drive to explore and the aversiveness of heights and open spaces of rats or mice. The maze is a cross made up of two open and two closed arms that is raised above the ground. The combination of light, the open arms, and the height is thought to produce unconditioned fear or anxiety responses in mice or rats. The test apparatus is an open top maze constructed of opaque plastic with alternating open and enclosed arms. For rats, each arm is 45-55 cm long and 8-12 cm wide, with the sides of the enclosed arms 35-45 cm high, the juncture approximately 10 x 10 cm, and the maze is elevated 45-55 cm above the floor. The mouse elevated plus maze consists of two closed arms (15 x 6 x 30 cm) and two open arms (1 x 6 x 30 cm) forming a cross, with a quadrangular center (6 x 6cm). The maze is placed 50 cm above the floor. Testing is performed in a room free of noise and distraction. On test days animals are administered drug or vehicle. If a pretreatment period is necessary, the animals are returned to the home cage for the duration of the pretreatment time; otherwise, the animals are placed in a clear plastic holding chamber singly or with cage mates for 1-10 minutes prior to test time. Rats are then placed in the center of the maze always oriented in the same direction, either consistently facing an open arm or an enclosed arm. For 5-10 minutes, entries into each arm and the time spent in each arm are recorded by the observer (s) or by videotape or a computer receiving input from a video camera mounted above the maze. To count as an entry, all four paws must be inside the arm. If necessary, additional measures of anxiety-related behaviors will be recorded,

i.e., time spent motionless, time spent in the center, time spent grooming, and the number of rears, stretching postures or feces produced. Following testing the animals are returned to the home cages. When animals are placed in the center of the maze, they spend most of their time in the closed arms, avoiding the open arms. Anxiolytic drugs, such as benzodiazepines, will increase the amount of time animals spend in the open arms. The test is also sensitive to anxiogenic drugs, which lends strong support for its predictive validity.

**Erectile Dysfunction**

Drugs affecting erectile function may be tested by measuring the effect on apomorphine-evoked increases in intracavernous pressure in the awake rat as described by Andersson, et al., (J. Urol. 161 : 1707-17] 2, 1999). One end of a polyethylene tubing is implanted into the cavernosal space of the penis of male Sprague-Dawley rats. After recovery from the surgery, intracavernous pressure is recorded using a pressure transducer connected to a multichannel pen-recorder. Erections are induced by administration of apomorphine (100-250 ug/kg s.c.) with or without test compound, and the results are compared for the treated group and the non-treated group.

**Female Sexual Dysfunction**

Systems to test compounds for the treatment of female sexual dysfunction include in vitro and in situ models using vaginal or clitoral smooth muscle preparations, histological evaluation, and vaginal blood flow assessments. In vivo studies of sexual responses focus on behavioral paradigms involving lordotic posturing and receptivity, as well as indices of motivation using a dual chamber pacing method (see, e.g., Hale, et al., Int. J. Impot. Res. 15 Suppl 5: S75-79, 2003).

The present invention is illustrated below with the aid of examples. These illustrations are given solely by way of example and do not limit the general spirit of the present invention.

**Examples:**

**Example 1:**

**N-piperidinyl-5-(4-chlorophenyl)-1-(2,4-dichlorophenyl)-4,5-dihydro-1H-pyrazole-3-carboxamide**

**a) 4-(4-chlorophenyl)-2-oxo-3-butenoic acid**

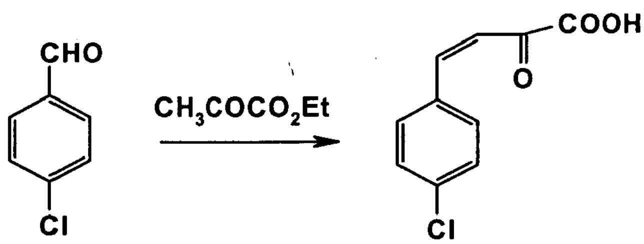


In a three neck flask p-chlorobenzaldehyde (13,3 g, 95 mmoles) and ethyl pyruvate (10 g, 86 mmoles) were dissolved in 150 ml of absolute ethanol.The solution was ice-cooled to 0°C and an aqueous solution of NaOH (3.8 g in 45 mL water) was added dropwise keeping the temperature below or equal to 10°C, whereby a yellow-orange colored precipitate was formed. The reaction mixture was stirred for 1 hour at 0°C and an additional 1.5 hours at room temperature (approximately 25 °C). Afterwards the reaction mixture was cooled down to approximately 5°C and the insoluble sodium salt of 4-(4-chlorophenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(4-chlorophenyl)-2-oxo-3-butenoic acid was then treated with a solution of 2N HCl, stirred for some minutes and solid 4-(4-chlorophenyl)-2-oxo-3-butenoic acid was separated via filtration and dried to give 12.7 g of the desired product (70% of theoretical yield).

IR (KBr, $cm^{-1}$): 3500-2500, 1719,3, 1686,5, 1603,4, 1587,8, 1081,9.
$^1H$ NMR($CDCl_3$, δ): 7,4 (d, J=8,4Hz, 2H), 7,5 (d, J=16,1Hz, 1H), 7,6 (d, J=8,4Hz, 2H), 8,1(d, J=16,1Hz, 1H).

**b) 5-(4-chlorophenyl)-1-(2,4-dichlorophenyl)-4,5-dihydro-pyrazole-3-carboxylic acid**

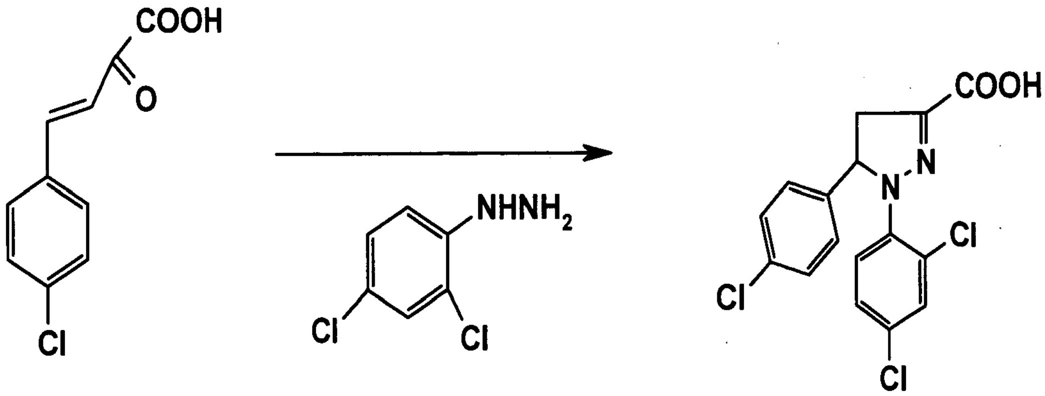

4-(4-chlorophenyl)-2-oxo-3-butenoic acid obtained according to step a) (12.6 g, 60 mmoles), 2,4-dichlorophenylhydrazine hydrochloride (12.8 g, 60 mmoles) and glacial acetic acid (200 mL) were mixed under a nitrogen atmosphere and heated to reflux for 4 hours, cooled down to room temperature (approximately 25 °C) and given into ice-water, whereby a sticky mass was obtained, which was extracted with methylene chloride. The combined methylene chloride fractions were washed with water, dried with sodium sulfate, filtered and evaporated to dryness to give a pale yellow solid (12.7 g, 57% of theoretical yield).

IR (KBr, $cm^{-1}$) : 3200-2200, 1668,4, 1458, 1251,4, 1104,8.

$^1H$ NMR ($CDCl_3$, δ): 3,3 (dd, 1 H), 3,7 (dd, 1 H), 5,9 (dd, 1 H), 7,09-7,25 (m, 7H).

**(c) 5-(4-chlorophenyl)-1-(2,4-dichlorophenyl)-4,5-dihydro-pyrazole-3-carboxylic acid chloride**

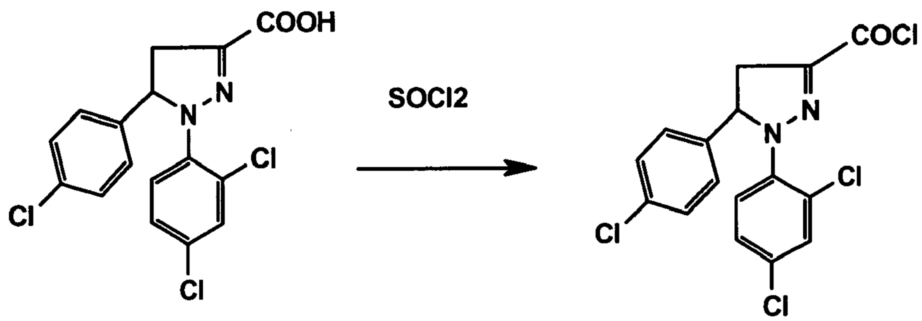


Under nitrogen atmosphere 5-(4-chlorophenyl)-1-(2,4-dichlorophenyl)-4,5-dihydro-pyrazole-3-carboxylic acid (2.5 g, 6.8 mmols) obtained according to step (b) was dissolved in 4 mL of in thionyl chloride and heated to reflux for 2.5 hours. The excess thionyl chloride is removed from the reaction mixture under reduced pressure and the resulting crude residue (2.6 g) is used without any further purification.

IR (KBr, $cm^{-1}$): 1732,3, 1700, 1533,3, 1478,1, 1212,9, 826,6.

**d) N-piperidinyl-5-(4-chlorophenyl)-1-(2,4-dichlorophenyl)-4,5-dihydro-1H-pyrazole-3-carboxamide** [this compound may also be referred to as 5-(4-Chloro-phenyl)-1-(2,4-dichlorophenyl)-4,5-dihydro-1H-pyrazole-3-carboxylic acid piperidin-1-ylamide or as 1-(2,4-dichlorophenyl)-5-(4-chlorophenyl)-4,5-dihydro-N-(piperidin-1-yl)-1H-pyrazole-3-carboxamide, or ]

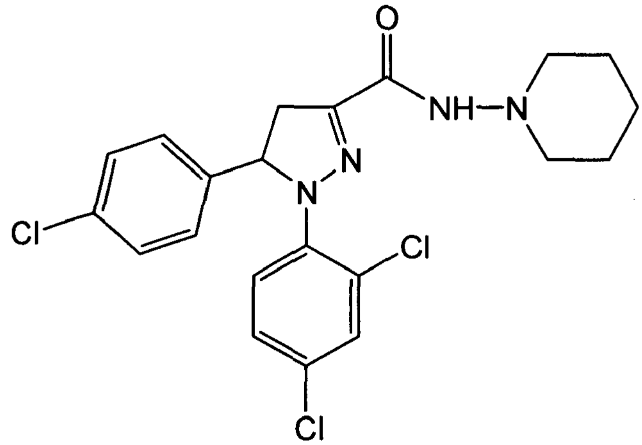

Under nitrogen atmosphere N-aminopiperidine (0.6 mL, 5.6 mmoles) and triethylamine (4 mL) were dissolved in methylene chloride (25 mL). The resulting mixture was ice-cooled down to 0°C and a solution of 5-(4-chlorophenyl)-1-(2,4-dichlorophenyl)-4,5-dihydropyrazole-3-carboxylic acid chloride obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give a total amount of 1.7 g (57% of theoretical yield) of N-piperidinyl-5-(4-chlorophenyl)-1-(2,4-dichlorophenyl)-4,5-dihydropyrazole-3-carboxamide having a melting point of 183-186°C.

IR (KBr, $cm^{-1}$): 3222,9, 2934,9, 1647,4, 1474,7, 1268,3, 815,6.

$^1H$ NMR ($CDCl_3$, δ) : 1,4 (m, 2H), 1,7 (m, 4H), 2,8 (m, 4H), 3,3 (dd, J=6,1 y 18,3Hz, 1H), 3,7 (dd, J=12,5 and 18,3 Hz, 1 H), 5,7 (dd, J=6,1 and 12,5 Hz, 1 H), 7,0-7,2 (m, 6H), 7,4 (s, 1H).

**Example 2-1:**

**α-Polymorph of N-piperidinyl-5-(4-chlorophenyl)-1-(2,4-dichlorophenyl)-4,5-dihydro-1H-pyrazole-3-carboxamide**

The α-Polymorph was obtained by evaporation of a solution of the compound according to example 1 (N-piperidinyl-5-(4-chlorophenyl)-1-(2,4-dichlorophenyl)-4,5-dihydro-1H-pyrazole-3-carboxamide) in ethanol at room temperature (25°C).

**Example 2-2:**

**α-Polymorph of N-piperidinyl-5-(4-chlorophenyl)-1-(2,4-dichlorophenyl)-4,5-dihydro-1 H-pyrazole-3-carboxamide**

The α-Polymorph was obtained by evaporation of a solution of the compound according to example 1 (N-piperidinyl-5-(4-chlorophenyl)-1-(2,4-dichlorophenyl)-4,5-dihydro-1H-pyrazole-3-carboxamide) in methanol at room temperature (25°C).

**Example 2a:**

**Crystal structure of the α-Polymorph of N-piperidinyl-5-(4-chlorophenyl)-1-(2,4-dichlorophenyl)-4,5-dihydro-1H-pyrazole-3-carboxamide**

Crystals suitable for X-ray single crystal determination could be optained from methanol and ethanol (also dimethylformamide) by slow evaporation at room temperature. Typically the α-modification (α-polymorph) grows in form of twinned needles.

A selected crystal was tested using an X-ray single crystal structure determination.

**Procedure:**

The measured crystals were selected using a Zeiss stereomicroscope using polarized light and prepared under inert conditions immersed in perfloropolyether as protecting oil for manipulation. Crystal structure determination was carried out using a Bruker-Nonius diffractometer equipped with a APPEX 2 4K CCD area detector, a FR591 rotating anode with $Mo_{k\alpha}$ radiation, Montel mirrors as monochromator and a Kyroflex low temperature device (T = 100K). Fullsphere data collection omega and phi scans.Analysis was done with software.
The α-Polymorph crystallizes in a triclinic cell.
A summary of the cell constants is given in table 1 below:

| | Unit cell dimensions | |
|---|---|---|
| Triclinic $P_1$ | a = 9.6749(11) Å | α = 78.112(3)°. |
| Volume: 2121.3(4) Å$^3$ | b = 13.1273(17) Å | β = 74.449(3)° |
| Density: 1.415 Mg/m$^3$ | C = 17.725 (2) Å | γ = 88.447(3)° |

**Example 2b:**

**DSC analysis of α-Polymorph of N-piperidinyl-5-(4-chlorophenyl)-1-(2,4-dichlorophenyl)-4,5-dihydro-1H-pyrazole-3-carboxamide**

Differential Scanning calorimetry (DSC) analyses were recorded in Mettler Toledo DSC822e. Samples of approximately 2 mg were weighted into 40 μl aluminium crucibles with a pinhole lid, and were submitted, under nitrogen (50 ml/min), to the temperature program.
The DSC analysis of the α-Polymorph presents a sharp melting peak with an onset of 183-184 °C and a maximum at 184-186 °C. The melting enthalpy is -87 ± 4 J/g, though this is slightly varying between samples.
**The following table shows the different melting peaks:**

| | **Onset** | **Peak** | **$\Delta H_f$** |
|---|---|---|---|
| **Sample 1** | 184°C | 185°C | -90 J/g |
| **Sample 2** | 184°C | 186°C | -87 J/g |
| **Sample 3** | 183°C | 185°C | -83 J/g |
| **Sample 4** | 183°C | 184°C | -84 J/g |

**Example 2c:**

**TG analysis of the α-Polymorph of N-piperidinyl-5-(4-chlorophenyl)-1-(2,4-dichlorophenyl)-4,5-dihydro-1H-pyrazole-3-carboxamide**

Thermogravimetric analyses (TGA) were recorded in a Mettler Toldo SDTA851 e. Samples of approximately 2 mg were weighted into open 40 μlalumina crucibles, and heated at 10°C/min between 30 and 600°C, under nitrogen (80 ml/min).
The TG analysis of the α-Polymorph presents weight loss is observed at temperatures higher than 280°C due to decomposition, and no weight loss is observed by lower temperatures.

**Example 2d:**

**FTIR Spectrum of the α-Polymorph of N-piperidinyl-5-(4-chlorophenyl)-1-(2,4-dichlorophenyl)-4,5-dihydro-1H-pyrazole-3-carboxamide**

FTIR spectra were recorded using a Bruker Tensor 27, equipped with a MKII golden gate single reflection ATR system, a mid-infrared source as the excitation source and a DTGS detector. All spectra were acquired at aresolution of 2 $cm^{-1}$ and 64 scans. No sample preparation was required to perform the analysis.
A representive example of the FTIR Spektrum of the α-Polymorph is shown in Figure 10.

**Example 2e:**

**FTRaman spectrum of the α-Polymorph of N-piperidinyl-5-(4-chlorophenyl)-1-(2,4-dichlorophenyl)-4,5-dihydro-1H-pyrazole-3-carboxamide**

FTRaman spectra were recorded using a Thermo Nicolet NXR FTRaman spectrometer with a near inrered Nd-YVO4 laser (1064 nm) at 200 mW as the excitation source and a germanium detector. All spectra were acquired at a resolution of 4 $cm^{-1}$ and 32 scans, and a 180° backscattering collection mode was employed. Sample was placed in a 1.6 mm external diameter glass capillary tube and were analyzed without further manipulation.

The FTRaman spectrum of the α-Polymorph shows intense bands at 2932,1647, 1593, 1394, 1269, 1253, 1109, 1084, 1047, 783, and 661 $cm^{-1}$. The spectrum presents Raman shifts around 1253, 1084, 533 and 364 $cm^{-1}$.

**Example 2f: Standard X-Ray Powder diffraction pattern of the α-Polymorph of N-piperidinyl-5-(4-chlorophenyl)-1-(2,4-dichlorophenyl)-4,5-dihydro-1H-pyrazole-3-carboxamide**

In order to acquire powder diffraction patterns of the obtained solids, approximately 20 mg of the non-manioulated samples were prepared in standard sample holders using to foils of polyacetate.
Powder diffraction patterns were acquired on a D8 Advance Series 2 Theta/Theta powder diffraction system using $Cu_{K\alpha}$-radiation in transmission geometry. The system is equipped with a Scintillation counter (NaI), a Germanium monochromator, a nine position sample stage, fixed divergence slits and a secondary soller slit. Programs used: Data collection with DIFFRAC plus XRD Commander V.2.4.1 and evaluation with EVA V.9.0.0.2.

For a representative sample see **Figure 8**). The sample was also measured using a micronized sample of the α-Polymorph (**Figure 9**).

The table below gives a list of selected peaks obtained (d-Values in Å)

| d-Values in Å | relative intensity (%) | d-Values in Å | relative intensity (%) |
|---|---|---|---|
| 17.18 | 100 | 4.86 | 77 |
| 13.02 | 11 | 4.79 | 59 |
| 11.56 | 18 | 4.74 | 43 |
| 9.46 | 10 | 4.65 | 79 |
| 8.55 | 10 | 4.54 | 23 |
| 7.74 | 11 | 4.48 | 11 |
| 7.31 | 17 | 4.42 | 10 |
| 6.82 | 29 | 4.37 | 23 |
| 6.56 | 12 | 4.27 | 18 |
| 5.75 | 10 | 4.22 | 11 |
| 5.65 | 16 | 4.15 | 31 |
| 5.52 | 12 | 4.01 | 23 |
| 5.45 | 15 | 3.96 | 33 |
| 5.26 | 23 | 3.84 | 16 |
| 5.21 | 21 | 3.73 | 40 |

**Example 2g:**

**Behaviour of the α-Polymorph of N-piperidinyl-5-(4-chlorophenyl)-1-(2,4-dichlorophenyl)-4,5-dihydro-1H-pyrazole-3-carboxamide under pressure**

Samples of the a-Polymorph were submitted to pressure stress for various periods of time (1, 5 and 30 minutes) to investigate the effect of pressure on the solid form. The pressure (98kN) used was chosen to correspond to correspond to the pressure at which planar tablets, of a diameter between 10 and 15 mm, are made. The application of pressure

on the samples did not induce any phase change. No differences were observed for experiment times between 1 and 30 minutes.
Precisely, 13 mm diameter samples of the polymorph were submitted to 98.07 kN of pressure using a Specac press for 1, 5 and 30 minutes and analysed, with no difference.

**Pharmacological Data:**

**Pharmaceutical Example : Effect of COMPOUND C (Example 1 / (rac)-N-piperidinyl-5-(4-chlorophenyl)-1-(2,4-dichlorophenyl)-4,5-dihydro-1H-pyrazole-3-carboxamide) on glycaemic control and on other metabolic disturbances associated with the aetiology of impaired glucose tolerance/insulin resistance and/or Type 2 diabetes.**

**METHODS**

**Experiment in male, dietary-induced (DIO) Sprague-Dawley rats**

The experiment was performed on selectively bred, male, Sprague-Dawley rats that were assessed at 3-5 weeks of age as having a predisposition to become obese. These animals were maintained on a normal light cycle (Lights on between 06.00h and 18.00h) under controlled temperature conditions. These rats were then given ad lib access to a commercial, energy-dense, high-fat diet. The animals for the study were 24 weeks old at the start of the experiment.
Groups of rats were injected intraperitoneally with COMPOUND C (10 mg/kg in 0.1 % Tween in saline; n = 15) or vehicle (0.1 % Tween in saline; n = 10). Animals were injected daily between 09.00h and 11.00h and food intake and body-weights were measured daily at the time of injection from Day-0.

**Oral glucose tolerance test**

On Day-21, groups of compound- and vehicle-treated mildly fasted rats were administered 2 g/kg glucose (as a 2g/ml solution) via a gastric tube placed into the stomach. Blood samples for plasma insulin determinations were taken at -30 min, 0, 15, 30, 60 and 120 min.

**Blood sampling**

Blood samples were taken from the tail veins of mildly fasted rats (restricted to 50% of their normal food intake in the 20h period before sampling) for the determination of plasma concentrations of triglycerides FFAs and insulin on Day-1 and Day-21.

**Determination of plasma lipid and insulin concentrations**

Plasma concentrations of triacylglycerol were determined using a standard automated enzymatic assay and FFAs were quantified using an acyl-CoA oxidase based colourimetric assay. Plasma insulin was determined by an ultra-sensitive ELISA method.

**Fat depots analysis**

On the day of termination of the experiment, inguinal, epidiymal, mesenterial and retroperitoneal fat depots were removed and weighed.

**Statistical analyses**

Calculations and statistical analyses were performed using GraphPad and Statview software packages. One-way ANOVA followed by Fischer's post hoc test was used to analyse the experimental data. $P < 0.05$ was set as the acceptance level for significant difference between groups.

## Experiment in female, dietary-induced (DIO), female Wistar rats

### Animals

The experiment was performed on female Wistar rats (originally in the weight range 250-300g). Animals were maintained on a reverse phase light-dark cycle (lights off for 8 h from 09.30-17.30 h) during which time the room was illuminated by red light. Animals had free access to powdered high fat diet (VRF1 plus 20% lard), ground chocolate, ground peanuts and tap water at all times. The three different diets were contained in separate glass feeding jars with aluminium lids with a 3-4 cm hole cut in it to allow access to the food. Animals were housed in pairs for twelve weeks for the induction of obesity. Animals were then housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

### Experimental procedures for the feeding study

Approximately a week before the start of the study, animals were weighed (to the nearest 0.1 g using an electronic top-pan balance) and allocated into 2 weight-matched treatment groups, each containing 10 animals. Following a 7 day baseline run-in period, during which the animals were dosed by the appropriate route (eg orally) once a day with vehicle (1% methylcellulose; 1 ml/kg) or the test drug, ie COMPOUND C (30 mg/kg po) once daily for 28 days.
Body weight was measured once daily.

### Plasma analyses

Terminal blood samples were taken via cardiac puncture from the animals immediately after death into Sarstedt 4.5 ml lithium-heparin tubes (Cat No. 32.331). Tubes were then centrifuged at 1,500 g for 5 minutes at 4°C and the plasma removed and immediately frozen on dry ice (as 3 aliquots per sample in sealed Eppendorf tubes). Plasma samples were stored at -75°C until required for analysis. All determinations were performed on plasma samples that had been thawed only once.

### Insulin assay

Plasma insulin concentration was determined using Mercodia ultrasensitive rat insulin ELISA kits according to the manufacturers 50 μl sample protocol. Unknown samples were diluted 20 times using the zero standard prior to assay in duplicate. Optical density at 450 nm was determined using a Molecular Devices VERSAmax tunable microplate reader and readings transferred to GraphPad Prism. A cubic spline curve was fitted to the calibration data and unknown values extrapolated from this curve fit.

### Glycerol and triacylglycerol assay

Plasma triacylglycerol was determined by modification of a discrete assay kit (Sigma Triglyceride determination kit, Cat No. TR0100) to a 96-well format. The kit actually determines liberated glycerol following enzymic hydrolysis of triacylglycerol to free fatty acids and glycerol (1 mole glycerol per mole of triacylglycerol). For this reason, correction for the glycerol concentration originally present in the plasma is required. Plasma samples were, therefore, assayed in the absence (free plasma glycerol) or presence (total triacylglycerol) of lipoprotein lipase. Subtraction of free glycerol from total triacylglycerol concentration then derives the true triacylglycerol concentration.
A 2.5 mg/ml triolein equivalent glycerol standard was obtained from Sigma. The supplied Sigma mM conversion factor for this batch was 1.13 (ie 2.83 mM glycerol). A 10 mg/ml glycerol stock solution was prepared in saline and then diluted in saline to create a series of standard concentrations from 0.218 to 4.34 mM. Standards and plasma samples (undiluted) were pipetted into the 96-well plates as 10 μl additions in duplicate. Assay plates and reagents were preheated at 37°C and the reaction started by adding 200 μl of either the glycerol or total triacylglycerol reagent (reconstituted as per manufacturer's protocol) followed by incubation at 37°C for 5 minutes after which optical density at 540 nm was determined using a Molecular Devices VERSAmax tunable microplate reader. Optical density readings were then transferred to GraphPad Prism and unknown values extrapolated from a linear regression fit to the standard curve. The assay was linear up to the highest glycerol standard employed (4.34 mM). Subtraction of the glycerol concentration in the plasma samples from the total triacylglycerol concentration derived the true triacylglycerol concentration.

**Leptin assay**

Plasma leptin concentration was determined using Assay Designs rat leptin enzyme immunometric assay kits (Cat No. 900-015) according to the manufacturer's regular protocol. Unknown samples were diluted 100 times using leptin assay buffer prior to assay in duplicate. Optical density at 450 nm was determined using a Molecular Devices VERSAmax tunable microplate reader and readings transferred to GraphPad Prism. A second order polynomial curve was fitted to the calibration data and unknown values extrapolated from this curve fit.

**Determination of carcass fat content**

Total ether-extractable fat in the freeze-dried samples was estimated by the Soxhlet principle using a Foss Soxtec HT2 system. The protocol employed was based on the manufacturer's recommendations for meat samples, although freeze-dried samples obviated the necessity for either a fat hydrolysis step or mixing the sample with sand to ensure efficient extraction.

Samples of approximately 1 g were accurately weighed into 26 mm x 60 mm cellulose extraction thimbles which were then plugged with approximately 0.8 g cotton wool. Sample fat was extracted into pre-weighed (including a few boiling chips) standard metal extraction cups using 70 ml of petroleum ether at 40-60°C, a circulator temperature of 115°C and a cooling flow rate of 2 l/min. A boiling stage of 25 min was employed, followed by a 40 min wash stage. A 10 min drying stage sufficed to remove ether from the extraction cups.

Percentage fat in the freeze-dried sample was calculated as follows:

$$\% \text{ fat} = [100 \times (\text{final cup weight} - \text{initial cup weight})]/\text{sample weight}$$

Percentage fat in the original sample was calculated using the % water as follows:

$$\% \text{ fat in original sample} = \% \text{ fat} \times [(100 - \% \text{ water})/100]$$

**Determination of carcass protein content**

Total protein in the freeze-dried samples was estimated using the Kjeldahl principle. A micro-Kjeldahl procedure was performed using a Foss 2012 digestion block and a Foss 2200 distilling unit. The protocol used was based on the manufacturer's recommendations for meat samples.

Approximately 0.3 g of freeze-dried sample was accurately weighed into 100 ml digestion tubes (Foss UK Ltd) in racks of 12 tubes. Each rack included at least one blank tube to determine a reagent blank. To each tube was added two Kjeltabs CQ and one Antifoam S tablet followed by 10 ml of 98% sulphuric acid. Samples were digested at 420°C for 1 h to convert protein into ammonium sulphate. Once tubes had cooled, free ammonia was liberated by the addition of an excess of 40% NaOH and steam distilled into 30 ml of 4% boric acid indicator solution. Ammonia was then determined by titration against volumetric grade 0.1 M HCl using a Brand 25 ml digital burette. Nitrogen and protein content of the samples were calculated as follows:

$$\% \text{ nitrogen} = [(\text{sample titration} - \text{blank titration}) \times 0.1401]/\text{sample weight (g)}$$

$$\% \text{ protein} = \% \text{ nitrogen} \times 6.25$$

**Determination of carcass ash content**

Total ash in the freeze-dried samples was estimated by determining the non-combustible ash remaining after firing samples at high temperatures using a Carbolite OAF 11/1 muffle furnace.

Squat form silica crucibles were prefired (600°C) for at least 2 h to stabilise weights. Crucibles were allowed to cool (<300°C) and transferred to silica gel drying cabinets. Crucibles were then weighed, approximately 1 g of sample added and the crucible reweighed. A number of empty crucibles were also included. Crucibles were fired at 600°C for 4 h to convert samples to ash and then allowed to cool (<300°C) prior to transfer to silica gel drying cabinets. Final

weights were determined when crucibles had fully cooled.

Percentage ash was determined by expressing the residual sample weight after firing as a percentage of the original sample weight. The average variation in blank crucible weight was -0.02 ± 0.04 mg (mean ± SEM, n=14) and individual weights varied by 0.5 mg or less.

**Experiment in growing, male Wistar rats**

Adult, male, Wistar rats aged between 11 and 12 weeks and weighing 250-280 g were used in the experiment. After 5 days of quarantine, they were housed initially in groups of 5, but were individually housed for use in the experiment. The animals were given free access to normal laboratory rat chow. The rats were maintained on a normal 12h -12h dark-light cycle and were kept in a vivarium at 18 - 24°C and 37 - 70% humidity.

After 2 weeks of acclimatization when food and water intake and bodyweights were measured on a daily basis, groups of rats were administered COMPOUND C (10 mg/kg or 30 mg/kg as a suspension in 5% gum arabic in saline; n = 15) or vehicle (5% gum arabic in saline; n = 15) by intragastric gavage. Animals were administered drug or vehicle once-daily for 14 days and food intake and body weights were measured daily at the time of injection.

**Blood sampling**

On Day-15, terminal blood samples were taken from 5 rats from each group for the determination of plasma concentrations of total cholesterol and triglycerides.

**Determination of plasma lipid and concentrations**

Plasma concentrations of total cholesterol and triacylglycerol were determined using a standard analyses performed in a Cardio Check™ Cardio reflectance photometer

**Statistical analyses**

Calculations and statistical analyses were performed using GraphPad and Statview software packages. Two-way ANOVA was used to analyse the body weight data. The total cholesterol, triglycerides and glucose concentration results were analysed by means of ANOVA RM followed by Dunnett's test. $P < 0.05$ was set as the acceptance level for significant difference between groups.

**RESULTS**

**Effects of COMPOUND C on glycaemic control**

Effects of COMPOUND C (10 mg/kg ip) on plasma insulin concentrations and responsiveness to an oral glucose challenge in male, DIO, Sprague-Dawley rats

Chronic administration of COMPOUND C improved the glycaemic profiles of these obese rats by statistically significantly ($p < 0.001$) reducing plasma insulin levels compared to the vehicle-treated control group (Figure 1).

When these rats were subjected to an oral glucose challenge test, the area under the curve (AUC) for the insulin excursion was significantly lower in the obese rats repeatedly given COMPOUND C than those given vehicle (Figure 2).

Effects of COMPOUND C (30 mg/kg po) on plasma insulin concentrations in female, DIO Wistar rats

Chronic administration of COMPOUND C improved the glycaemic profiles of these obese rats by reducing plasma insulin levels compared to the vehicle-treated control group (Figure 3).

**Effects of COMPOUND C on plasma lipid concentrations**

Effects of COMPOUND C (10 ma/kq ip) on plasma triglycerides and free fatty acid (FFA) concentrations in male, DIO, Sprague-Dawley rats

Chronic administration of COMPOUND C improved the lipid profiles of these obese rats statistically significantly ($p < 0.05$), reducing plasma trigyceride levels and FFA levels compared to the vehicle-treated control group (Figure 4).

Effects of COMPOUND C (30 mg/kg po) on plasma glycerol and triacylgycerol concentrations in female, DIO Wistar rats

Chronic administration of COMPOUND C significantly improved the lipid profiles of these obese rats by reducing plasma glycerol levels (by 23.4%) and triacylglycerol levels (by 37.2%) compared to the vehicle-treated control group (Figure 5).

Chronic effects of COMPOUND C (10 and 30 mg/kg po) on plasma triglyceride and cholesterol and concentrations in growing, male Wistar rats

Chronic administration of COMPOUND C (10 and 30 mg/kg po) dose-dependently improved the lipid profiles of growing, Sprague-Dawley rats by reducing plasma triglyceride levels compared to the vehicle-treated control group (Figure 6). The reduction was statistically significantly ($p < 0.05$) at the higher dose of COMPOUND C (Figure 6). COMPOUND C also dose-dependently reduced plasma cholesterol levels compared to the vehicle-treated control group (Figure 7). The reduction was statistically significantly ($p < 0.05$) at the higher dose of COMPOUND C (Figure 7).

Effect of COMPOUND C (10 mg/kg ip) on the visceral fat-pad weights of male, DIO, Sprague-Dawley rats

Consistent with a marked reduction in overall adiposity as shown by the decrease of bodyweight (Figure 8), measurement of the weights of the major visceral fat-pads, ie inguinal, epididymal, retroperitoneal and mesenterial, similarly revealed large reductions (Figure 11). These decreases were statistically significant for all of the visceral fat depots, except the epididymal pads (Figure 11).

Effects of COMPOUND C (30 mg/kg po) on the body composition of female, DIO Wistar rats

The body composition of the vehicle-treated group of animals was 49.0 ± 1.0% water, 31.9 ± 1.3% fat, 15.5 ± 0.3% protein and 2.77 ± 0.09% ash (total of 99.2%). The final mean carcass weight of the vehicle-treated control group was 473.0 ± 8.1 g. COMPOUND C significantly reduced final carcass weight by 72.8 g.

Chronic administration of COMPOUND C significantly reduced total carcass water by 11.8 g when compared to the vehicle-treated control. As the total carcass water content reduced (in proportion) by much less than the reduction in carcass weight, the percentage water content of the carcasses was actually significantly increased in the COMPOUND C-treated animals (from 49.0% to 54.3% Figure 12). COMPOUND C significantly and substantially reduced total carcass fat and percentage fat. Mean body fat mass was significantly reduced from 156.3 g in the vehicle group to 98.6 g following COMPOUND C treatment. Expressed as a percentage of carcass weight, fat content was significantly reduced from 31.9% in the vehicle-control group to 24.2% in the COMPOUND C -treated group (Figure 12). Thus, COMPOUND C produced a decrease in absolute body fat of 57.7 g compared to the vehicle-treated control group, representing 79.3% of the total weight-loss observed. This was equivalent to a marked 36.9% decrease in fat mass compared to the vehicle-treated control group. Percentage protein content was significantly increased from 15.5% in the vehicle-treated group to 17.1% in the COMPOUND C-treated group (Figure 12). COMPOUND C did not significantly alter total ash content, but significantly increased percentage ash content from 2.77% to 3.26% (Figure 12).

Effects of COMPOUND C (30 mg/kg po) on plasma leptin concentrations in female, DIO Wistar rats

Consistent with a marked reduction in the white adipose tissue mass, plasma leptin concentrations were markedly and significantly reduced following COMPOUND C (-48.2%) treatment compared to the vehicle-treated control group (Figure 13).

## Claims

1. α-Polymorph of (rac)-N-piperidinyl-5-(4-chlorophenyl)-1-(2,4-dichlorophenyl)-4,5-dihydro-1H-pyrazole-3-carboxamide.

2. α-Polymorph of (rac)-N-piperidinyl-5-(4-chlorophenyl)-1-(2,4-dichlorophenyl)-4,5-dihydro-1H-pyrazole-3-carboxamide, **characterized in that** it crystallizes as a triclinic cell with an α-angle of 78° and/or a β-angle of 74° and/or a γ-angle of 88°.

3. α-Polymorph of (rac)-N-piperidinyl-5-(4-chlorophenyl)-1-(2,4-dichlorophenyl)-4,5-dihydro-1H-pyrazole-3-carboxamide, **characterized in that** it shows a standard X-Ray powder diffraction according to figure 8.

4. α-Polymorph of (rac)-N-piperidinyl-5-(4-chlorophenyl)-1-(2,4-dichlorophenyl)-4,5-dihydro-1 H-pyrazole-3-carboxamide according to claim 1, **characterized in that** the α-Polymorph melts
above 180°C, preferably at an onset point of 183 ± 1 °C, more preferably at an onset point of 183-184 °C and/or
with a melting peak at 185 ± 2 °C, preferably at 184 ± 1 °C.

5. α-Polymorph of (rac)-N-piperidinyl-5-(4-chlorophenyl)-1-(2,4-dichlorophenyl)-4,5-dihydro-1 H-pyrazole-3-carboxamide according to claim 1, **characterized in that** α-Polymorph decomposes at or above 280 °C.

6. α-Polymorph of (rac)-N-piperidinyl-5-(4-chlorophenyl)-1-(2,4-dichlorophenyl)-4,5-dihydro-1H-pyrazole-3-carboxamide according to claim 1, **characterized in that** the α-Polymorph shows in the FTRaman spectrum bands, preferably intense bands, at 2932 ± 5 $cm^{-1}$ and/or 1647 ± 5 $cm^{-1}$ and/or 1593 ± 5 $cm^{-1}$ and/or 1394 ± 5 $cm^{-1}$ and/or 1269 ± 5 $cm^{-1}$ and/or 1253 ± 5 $cm^{-1}$ and/or 1109 ± 5 $cm^{-1}$ and/or 1084 ± 5 $cm^{-1}$ and/or 1047 ± 5 $cm^{-1}$ and/or 783 ± 5 $cm^{-1}$ and/or 661 ± 5 $cm^{-1}$ and/or a Raman shift at 1253 ± 5 $cm^{-1}$ and/or at 1084 ± 5 $cm^{-1}$ and/or at 533 ± 5 $cm^{-1}$ and/or and at 364 $cm^{-1}$

7. Process for the manufacture of the α-Polymorph of (rac)-N-piperidinyl-5-(4-chlorophenyl)-1-(2,4-dichlorophenyl)-4,5-dihydro-1H-pyrazole-3-carboxamide according to any of claims 1 to 6, **characterized in that** a solution of (rac)-N-piperidinyl-5-(4-chlorophenyl)-1-(2,4-dichlorophenyl)-4,5-dihydro-1H-pyrazole-3-carboxamide in an alcohol; preferably ethanol or methanol; is evaporated at room temperature (25 °C) to yield the α-Polymorph of (rac)-N-piperidinyl-5-(4-chlorophenyl)-1-(2,4-dichlorophenyl)-4,5-dihydro-1H-pyrazole-3-carboxamide

8. Medicament comprising at least one α-Polymorph of (rac)-N-piperidinyl-5-(4-chlorophenyl)-1-(2,4-dichlorophenyl)-4,5-dihydro-1H-pyrazole-3-carboxamide according to any of claims 1 to 6 and optionally one or more pharmaceutically acceptable excipients.

9. Use of the α-Polymorph of (rac)-N-piperidinyl-5-(4-chlorophenyl)-1-(2,4-dichlorophenyl)-4,5-dihydro-1H-pyrazole-3-carboxamide according to any of claim 1 to 6 for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

10. Use of the α-Polymorph of (rac)-N-piperidinyl-5-(4-chlorophenyl)-1-(2,4-dichlorophenyl)-4,5-dihydro-1H-pyrazole-3-carboxamide according to any of claims 1 to 6 for the manufacture of a medicament for the treatment of dyslipidemia or metabolic syndrome.

Figure 1 Effect of chronic administration of COMPOUND C 10 mg/kg ip on plasma insulin in male, dietary-induced obese, Sprague-Dawley rats

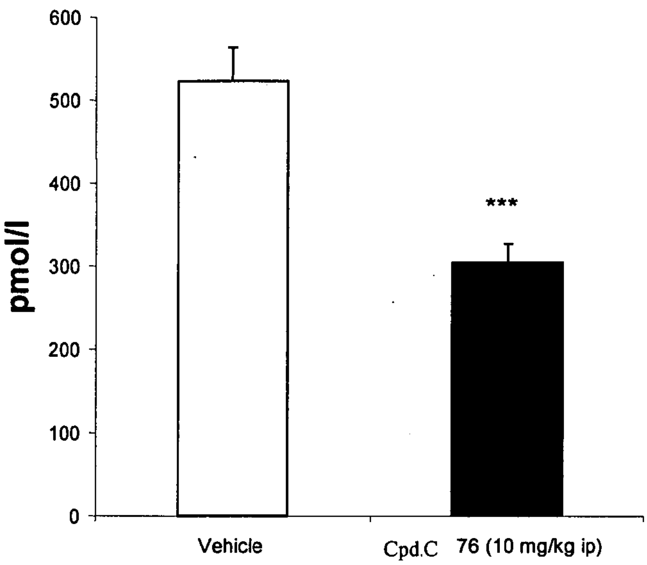


ANOVA factorial, Fisher's post-hoc test
***p<0,001 vs.

Figure 2 Effect of chronic administration of COMPOUND C 10 mg/kg ip in an oral glucose tolerance test in male, dietary-induced obese, Sprague-Dawley rats

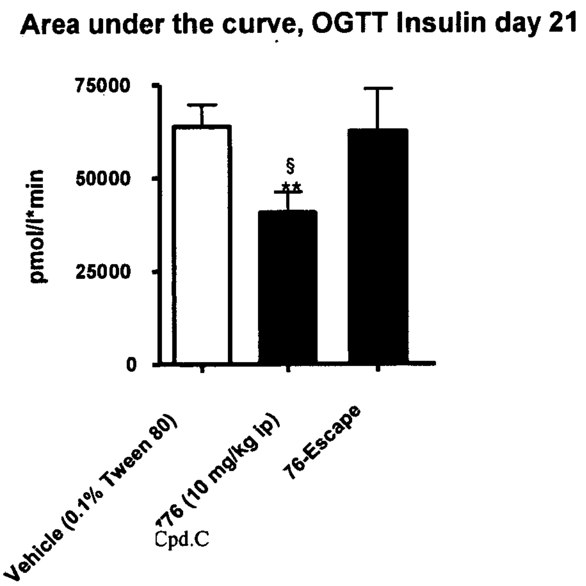

Figure 3 Effect of chronic administration of COMPOUND C 30 mg/kg po on plasma insulin levels in dietary-induced obese, female Wistar rats

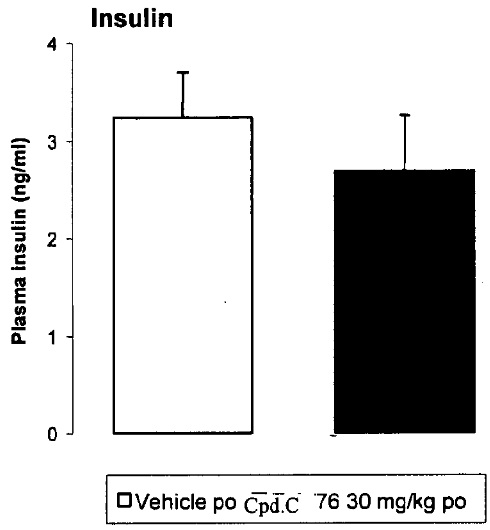


Results are expressed as treatment group means (adjusted for bleeding order and for differences between the groups in body weight at baseline (Day 1)) + SEM (calculated from the residuals of the statistical model); n = 10. Multiple comparisons against the vehicle-treated control group were determined by a multiple t test.

Figure 4 Effect of chronic administration of COMPOUND C 10 mg/kg ip on plasma triglycerides and free fatty acid levels in male, dietary-induced obese, Sprague-Dawley rats

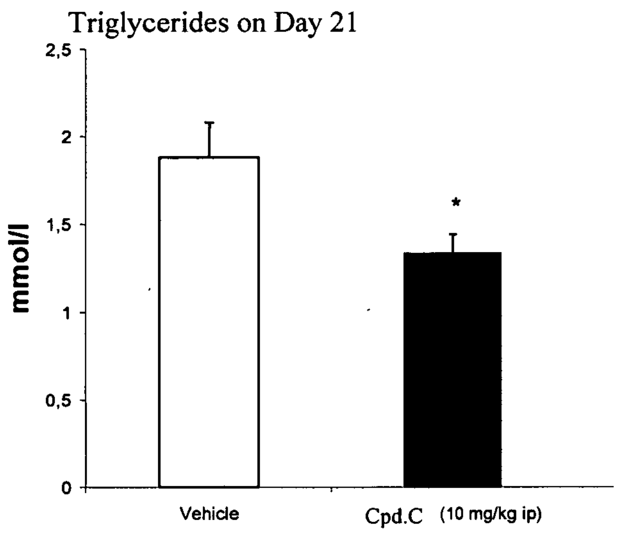


ANOVA factorial, Fisher's post-hoc test
* p<0,05 vs. vehicle

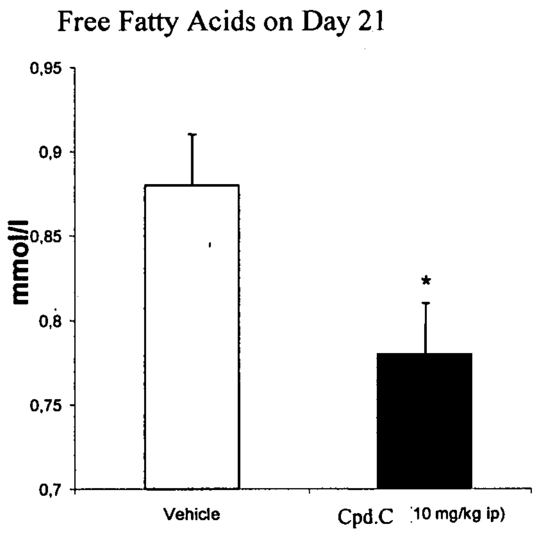


ANOVA factorial, Fisher's post-hoc test
* p<0,05 vs. vehicle

Figure 5 Effect of chronic administration of COMPOUND C on plasma glycerol and triacyglycerol levels in dietary-induced obese, female Wistar rats

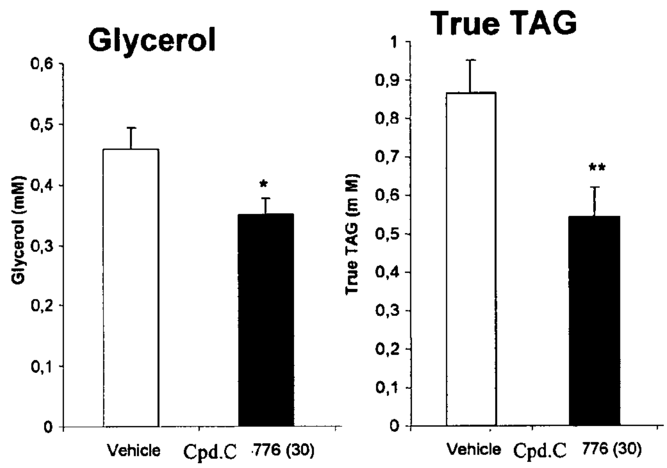


Results are expressed as treatment group means (adjusted for bleeding order and for differences between the groups in body weight at baseline (Day 1)) + SEM (calculated from the residuals of the statistical model); n = 10. Multiple comparisons against the vehicle-treated control group were determined by a multiple t test. Significant differences from the control group are denoted by *p<0.05 and **p<0.01.

Figure 6 Effect of COMPOUND C 10 and 30 mg/kg po on blood levels of triglycerides in growing male Wistar rats after 14 days of treatment

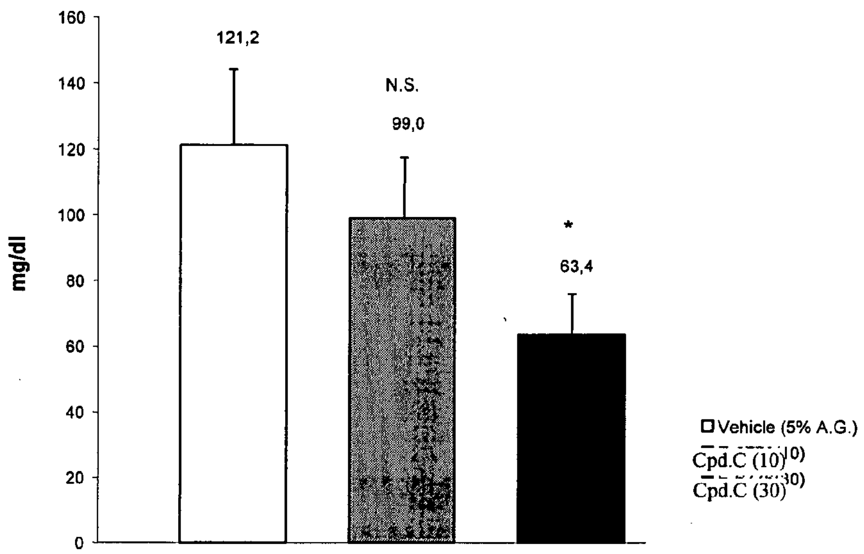

Figure 7 Effect of COMPOUND C 10 and 30 mg/kg po on blood levels of total cholesterol in male Wistar rats after 14 days of treatment

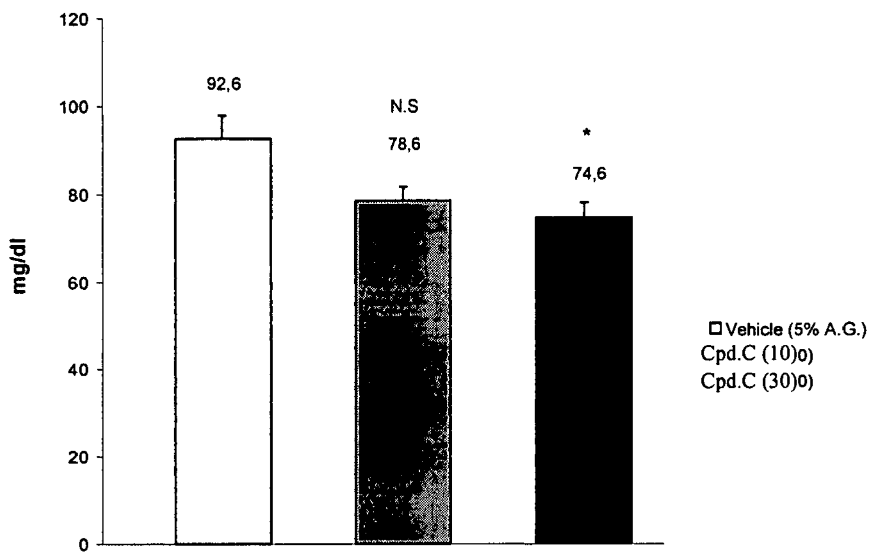

Figure 11 Effect of COMPOUND C 10 mg/kg ip on the fat pad depots in male, dietary-induced obese, Sprague-Dawley rats

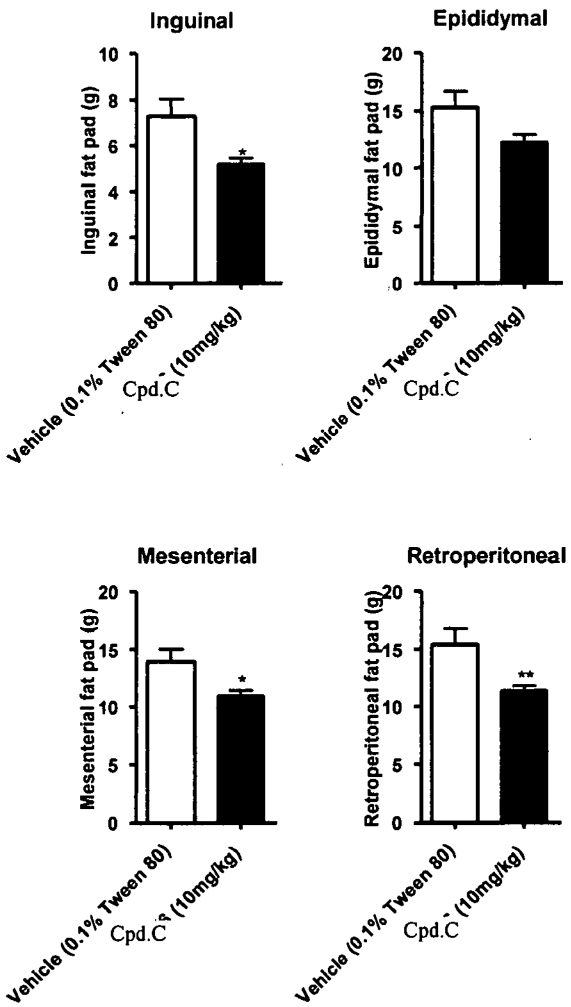

Figure 12 Effect of chronic administration of COMPOUND C 30 mg/kg po on carcass composition

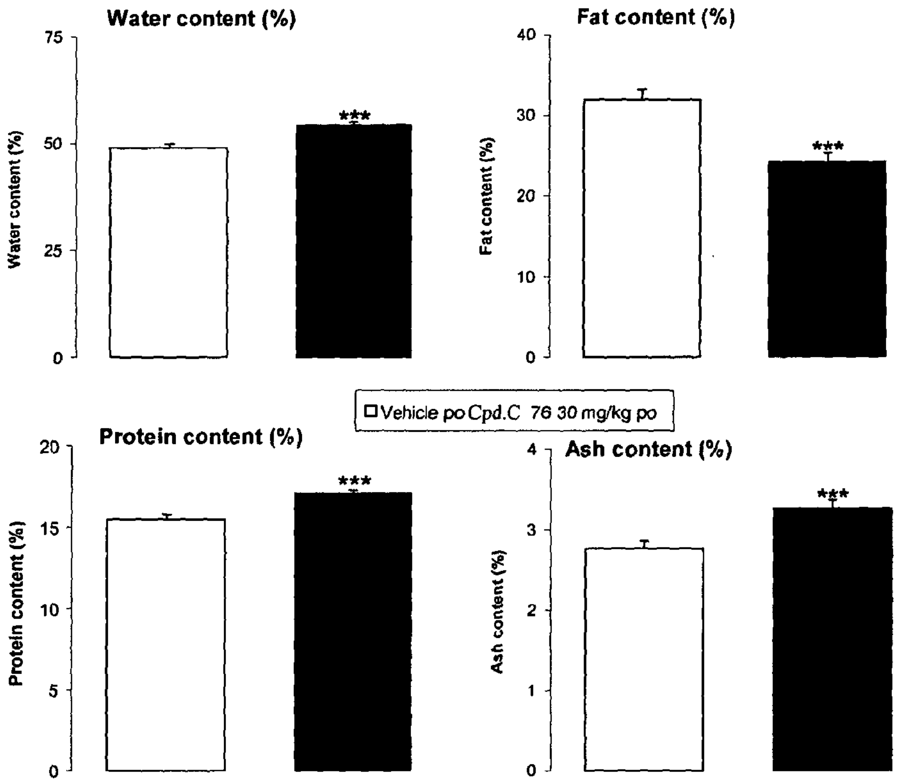


Data are expressed as percentage of final carcass weight.
Results are expressed as treatment group means (adjusted for differences between the groups in body weight at baseline (Day 1)) + SEM (calculated from the residuals of the statistical model); n = 10.
Multiple comparisons against the vehicle-treated control group were determined by a multiple t test Significant differences from the control group are denoted by ***$p<0.001$.

Figure 13 Effect of chronic administration of COMPOUND C 30 mg/kg po plasma leptin levels in dietary-induced obese, female Wistar rats

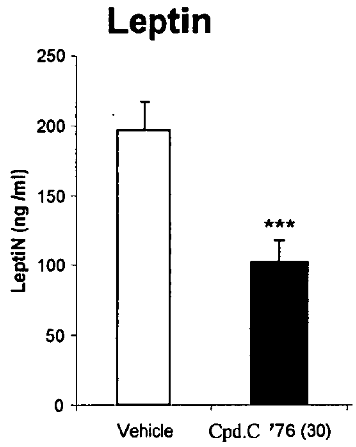


Results are expressed as treatment group means (adjusted for bleeding order and for differences between the groups in body weight at baseline (Day 1)) + SEM (calculated from the residuals of the statistical model); n = 10. Multiple comparisons against the vehicle-treated control group were determined by a multiple t test. Significant differences from the control group are denoted by ***$p<0.001$.

**Figure 8:** Standard X-ray powder diffraction pattern of α-modification.

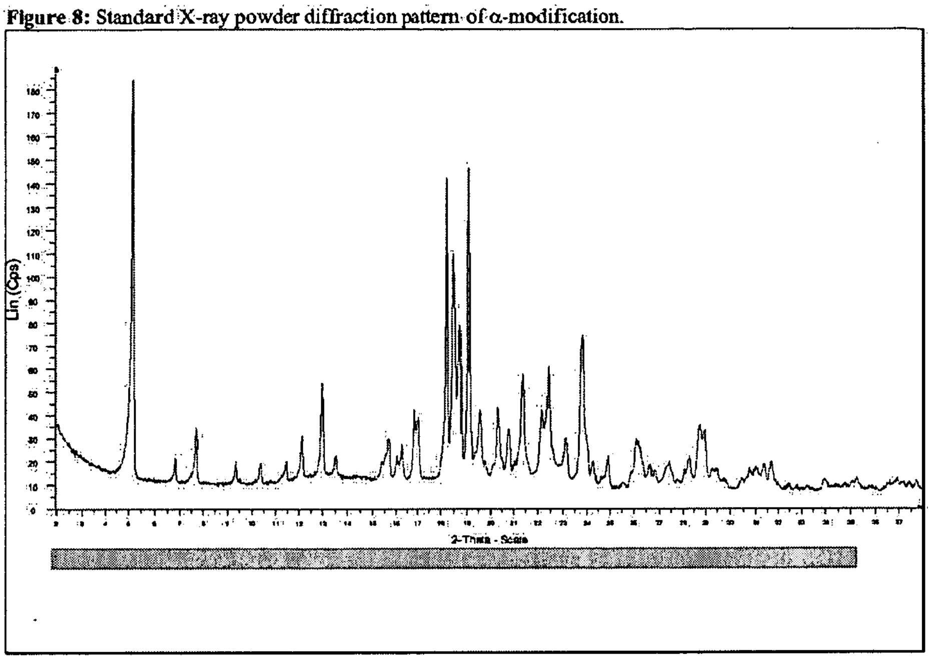


**Figure 9:** X-ray powder diffraction pattern of a micronized sample of α-modification.

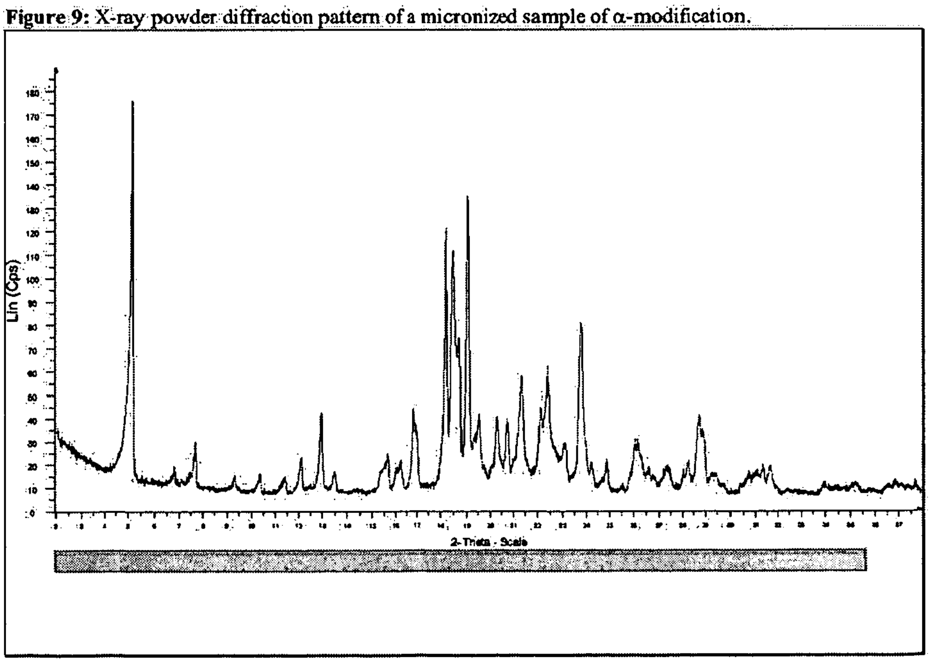

Figure 10 : FTIR analysis of modification α

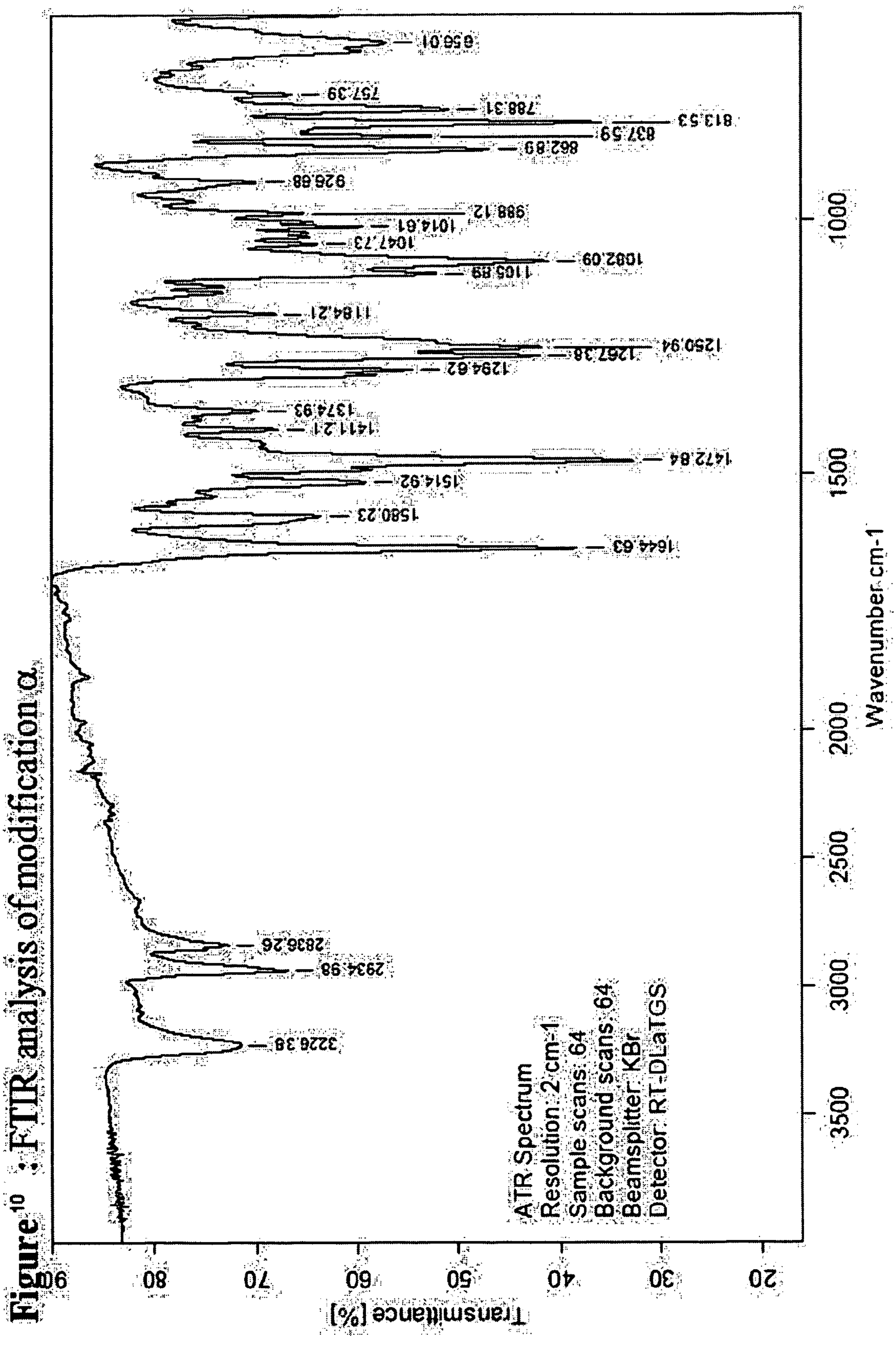

European Patent Office

# EUROPEAN SEARCH REPORT

Application Number
EP 07 00 1041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 1 757 588 A (ESTEVE LABOR DR [ES]) 28 February 2007 (2007-02-28) * the whole document * ----- | 1-10 | INV. C07D231/06 A61K31/4155 A61P3/04 A61P3/10 |
| D,A | WO 2005/077911 A (ESTEVE LABOR DR [ES]; CUBERES ALTISEN ROSA [ES]; FRIGOLA CONSTANSA JOR) 25 August 2005 (2005-08-25) * example 1 * ----- | 1-10 | |

TECHNICAL FIELDS SEARCHED (IPC)
C07D

The present search report has been drawn up for all claims

| Place of search | Date of completion of the search | Examiner |
|---|---|---|
| Munich | 14 May 2007 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 07 00 1041

This annex lists the patent family members relating to the patent documents cited in the above-mentioned European search report.
The members are as contained in the European Patent Office EDP file on
The European Patent Office is in no way liable for these particulars which are merely given for the purpose of information.

14-05-2007

| Patent document cited in search report | Publication date | Patent family member(s) | Publication date |
|---|---|---|---|
| EP 1757588 A | 28-02-2007 | NONE | |
| WO 2005077911 A | 25-08-2005 | AR 051431 A1 | 17-01-2007 |
| | | AU 2005212836 A1 | 25-08-2005 |
| | | CA 2556400 A1 | 25-08-2005 |
| | | EP 1626963 A1 | 22-02-2006 |
| | | JP 2007507471 T | 29-03-2007 |
| | | KR 20060129055 A | 14-12-2006 |
| | | US 2006020010 A1 | 26-01-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005077911 A

### Non-patent literature cited in the description


- **HOLLISTER.** *Pharm. Rev.,* 1986, vol. 38, 1-20
- **RENY ; SINGHA.** *Prog. Drug. Res.,* 1991, vol. 36, 71-114
- **CONSROE ; SANDYK.** Neurobiology and Neurophysiology. CRC Press, 1992, vol. 459
- **ECKEL et al.** *The Lancet,* 2005, vol. 365, 1415-1428
- **ALBERTI et al.** *Diabet. Med.,* 1998, vol. 15, 539-53
- *JAMA,* 2001, vol. 285, 2486-97
- **PAGOTTO ; PASQUALI.** *The Lancet,* 2005, vol. 365, 1363, 1364
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672
- **GOVAERTS et al.** *Eur J Pharmac Sci,* 2004, vol. 23, 233-243
- **A. C. HOWLETT et al.** International Union of Pharmacology XXVII. *Classification of Cannabinoid Receptors, Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. - Forsch. (Frug Res,* 1975, vol. 25, 9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effects of Hoe 249: A new potential antidepressant. *Drugs Dev. Res.,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117
- **E.T. TZAVARA et al.** The CB1 receptor antagonist SR141716A selectively increases monoaminergic neurotransmission in the medial prefrontal cortex: implications for therapeutic actions. *Br. J. Pharmacol.,* 2003, vol. 138 (4), 544
- **LUMENG, L et al.** Different sensitivities to ethanol in alcohol-preferring and-nonpreferring rats. *Pharmacol, Biochem Behav.,* 1982, vol. 16, 125-130
- **MIDDAUGH et al.** Ethanol Consumption by C57BU6 Mice : Influence of Gender and Procedural Variables. *Alcohol,* 1999, vol. 17 (3), 175-183
- **LE et al.** Alcohol Consumption by C57BL/6, BALA/c, and DBA/2 Mice in a Limited AccessParadigm. *PharmacologyBiochemistry and Behavior,* 1994, vol. 47, 375-378
- **VASTOLA et al.** *Physiol. Behav.,* 2002, vol. 77, 107-114
- **BROWER et al.** *Brain Res.,* 2002, vol. 930, 12-20
- **PORSOLT et al.** *Nature,* 1977, vol. 266, 730-732
- **STEM et al.** *Psychopharmacology,* 1985, vol. 85, 367-370
- **SWERDLOW ; GEYER.** *Schizophrenia Bulletin,* 1998, vol. 24, 285-301
- **PELLOW ; FILE.** *Pharm. Biochem. Behav.,* 1986, vol. 24, 525-529
- **ANDERSSON et al.** *J. Urol.,* 1999, vol. 161 (2), 1707-17
- **HALE et al.** *Int. J. Impot. Res.,* 2003, vol. 15 (5), 75-79